# EUROPEAN PATENT APPLICATION

(11) **EP 2 565 263 A1**
(43) Date of publication of application: **06.03.2013**
(21) Application number: 10850541.3
(22) Date of filing: 29.07.2010
(51) Int. Cl.: C12N 5/00, C12N 5/02, C12N 5/07

(54) **CULTURE MEDIUM ADDITIVE AND USES THEREOF**

(30) Priority: 30.04.2010 CN 201010167062
(71) Applicant: Guangzhou Institute Of Biomedicine And Health, Chinese Academy Of Sciences, Guangzhou, Guangdong 510530 (CN)
(72) Inventor: PEI, Duanqing, Guangzhou Guangdong 510530 (CN); CHEN, Jiekai, Guangzhou Guangdong 510530 (CN); LIU, Jing, Guangzhou Guangdong 510530 (CN)
(74) Representative: Gulde Hengelhaupt Ziebig & Schneider
(86) International application number: PCT/CN2010/075551
(87) International publication number: WO 2011/134210

(57) **Abstract**

The present invention provides a chemically defined culture system, by which induced pluripotent stem (iPS) cells are obtained with high efficiency. The culture medium supplement of the present invention includes vitamin C and a glycogen synthase kinase-3 inhibitor; another culture medium supplement of the present invention further includes, in addition to vitamin C and the glycogen synthase kinase-3 inhibitor, vitamin B 12, insulin, a receptor tyrosine kinase, and an anti-oxidant; and the culture medium supplement of the present invention may further be a mixture of the above two culture medium supplements with a serum replacement cell growth promoter. The present invention further provides a complete culture medium for iPS cells, which is formed by one or more of a basal culture medium, serum, and a serum replacement supplement, and the above culture medium supplements, or formed only by the above culture medium supplements and a basal culture medium. The culture system of the present invention is a chemically defined culture system free of serum and contamination from animal source, by which the iPS cells are obtained with high efficiency. The culture system maintains the growth and proliferation of the cells in conversion of somatic cells to iPS cells in absence of feeder cells, significantly accelerates the iPS cell induction process, and greatly improves the induction efficiency of somatic cells into iPS cells.

## Description

### FIELD OF THE INVENTION

The present invention relates to the field of a cell culture medium, and more particularly to a culture medium supplement, which can be used to culture induced pluripotent stem (iPS) cells or mammalian cells.

### BACKGROUND OF THE INVENTION

In 2008, Japanese scientist Yamanaka and his coworkers successfully reprogrammed mouse fibroblasts into embryonic stem (ES) cells with the four transcription factors, Oct4, Kif4, Sox2, and c-Myc, and this technology is referred to as iPS cell technology. Since then, fibroblasts derived from human and other species such as rat, monkey and pig, are also successfully reprogrammed, and thus this technology is considered as an important breakthrough in regeneration medicine. At present, almost all the culture systems used in reprogramming of mice contain serum. Use of the serum culture system has the following disadvantages. 1. The reprogramming process is slow. Serum is confirmed to contain some substances inhibiting reprogramming, for example, TGF-b family growth factors. In addition, other unknown ingredients in serum may also very likely delay the reprogramming process. These confirmed or unconfirmed substances make the reprogramming efficiency extremely low. 2. The serum stability is different from batch to batch. The preparation progression of serum determines that the ingredients of each batch of serum are different, which very likely leads to the decrease of the experimental repeatability. 3. The uncertainty of the ingredients in serum sets obstacles in study of the reprogramming mechanism and in drug screening. Therefore, the ingredients of serum are complex, and various pathways favorable to or unfavorable to the iPS process may be activated, which causes great background noises to the study of the iPS cell mechanism, and decrease the sensitivity of the drug screening.

Researches suggest that the invention of knockout serum replacement (KOSR) serum-free culture medium is a great advance in cell culture. The system is completely free of serum, and most of the ingredients in the system are clear, so that the experimental repeatability is much improved. In contrast to serum, the system almost contains no differentiation factors, which is better for the maintaining of the self-renewal of the stem cells. Furthermore, a mixture of the system with serum exhibits a higher reprogramming efficiency than that exhibited when serum is used alone.

However, the ingredients in the system are still not absolutely clear, for example, the bovine serum globulin complex (Albumax) contains many unknown lipids, and the effect of these ingredients on reprogramming is still unknown. Moreover, KOSR cannot support the growth of the cells in the early stage of the reprogramming. In addition, in induction of mouse iPS cells, the reprogramming efficiency is not high enough even if KOSR is mixed with serum. Therefore, a chemically defined high-efficiency reprogramming system free of serum is very important for the study of the iPS cell mechanism, the drug screening, and the clinical use of iPS cells.

### SUMMARY OF THE INVENTION

In order to overcome the technical problems above, the present invention provides a chemically defined culture system, by which the iPS cells can be obtained with high efficiency. The culture system can maintain the growth and proliferation of the stem cells in absence of feeder cells, accelerate the iPS cell progression, and improve the iPS efficiency.

In order to achieve the above objectives, the following technical solution is employed.

The culture medium supplement of the present invention includes vitamin C and a glycogen synthase kinase-3 inhibitor.

The culture medium supplement of the present invention may further include, in addition to vitamin C and the glycogen synthase kinase-3 inhibitor, vitamin B12, insulin, a receptor tyrosine kinase, and an anti-oxidant.

In order to achieve the above objectives, the following technical solution is employed.

The vitamin C includes ascorbic acid and a derivative thereof, for example, sodium ascorbate salt, and a stable form of the vitamin C, i.e. 2-phospho-ascorbic acid. A preferred form is 2-phospho-ascorbic acid. A working concentration of vitamin C is, but not limited to, 0-100 µg/ml, and is preferably 50 µg/ml. A working concentration of vitamin B 12 is, but not limited to 0-2.8 µg/ml, and is preferably 1.4 µg/ml.

The insulin includes extracted and artificially synthesized recombinant insulin derived from various sources and having biological activity. The recombinant human insulin (SIGMA Inc.) is preferred, and a working concentration is, but not limited to, 0-50 µg/ml, and is preferably 20 µg/ml.

The glycogen synthase kinase-3 inhibitor includes at least one of Li⁺, Chir99021, BIO, and SB216763. Li⁺ is preferably in the form of LiCl, and a working concentration is without limitation in the range of 0-10 µg/ml, and is preferably 5 mmol/ml. The glycogen synthase kinase is a class of serine/threonine phosphorylase kinase, and is involved in regulation of many signaling pathways. An inhibitor thereof is preferably CHIR99021, and a working concentration is without limitation in the range of 0-12 µmol/ml, and is preferably 3 µmol/ml.

The receptor tyrosine kinase includes at least one of basic fibroblast growth factor (bFGF), epidermal growth factor (EGF), vascular endothelial growth factor (VEGF), platelet-derived growth factor (PDGF), insulin-like growth factor (IGF), and hepatocyte growth factor (HGF). The bFGF is preferred, and a working concentration is in the range of 0-100 ng/ml, and is preferably 5 ng/ml.

The anti-oxidant includes at least one of thiamine, superoxide dismutase (SOD), catalase, reduced glutathione, vitamin E, acetylated vitamin E, linoleic acid, Linolenic acid, and sodium selenite. A working concentration of thiamine is without limitation in the range of 0-36 µg/ml, and is preferably 9 µg/ml; a working concentration of superoxide dismutase is without limitation in the range of 0-10 µg/ml, and is preferably 2.5 µg/ml. A working concentration of the reduced glutathione is without limitation in the range of 0-6 µg/ml, and is preferably 1.5 µg/ml. A working concentration of vitamin E is without limitation in the range of 0-16 µg/ml, and is preferably 1 µg/ml. A working concentration of the acetylated vitamin E is without limitation in the range of 0-16 µg/ml, and is preferably 1 µg/ml. A working concentration of linoleic acid is without limitation in the range of 0-4 µg/ml, and is preferably 1 µg/ml. A working concentration of ethanolamine is without limitation in the range of 0-16 µg/ml, and is preferably 1 µg/ml.

The culture medium supplement of the present invention may be a mixture of the above two culture medium supplements respectively with a serum replacement cell growth promoter, in which the serum replacement cell growth promoter includes at least one of albumin hydrolyzate, transferrin, triiodothyronine, adrenalone, lipoic acid, ethanolamine, progesterone, putrescine, and vitamin A.

The albumin hydrolyzate is a hydrolyzed product of albumin, and is composed of amino acids and polypeptides, the specific ingredients are clear, and a use concentration is without limitation in the range of 0-10 mg/ml, and is preferably 1 mg/ml.

The transferrin includes a transferrin with and without iron ions, and is preferably a transferrin with iron ions, and a use concentration is without limitation in the range of 0-200 µg/ml, and is preferably 100 µg/ml.

A working concentration of the lipoic acid is without limitation in the range of 0-3.2 µg/ml, and is preferably 0.2 µg/ml.

A working concentration of the vitamin A is without limitation in the range of 0-1.6 µg/ml, and is preferably 0.1 µg/ml.

The present invention further provides a complete culture medium, which is formed by one or more of a basal culture medium, serum, and a serum replacement supplement, and the above several culture medium supplements; or formed by a basal culture medium and the above several culture medium supplements. Meanwhile, ingredients are as configured in Table 1 and added to a basal culture medium (Dulbecco's Modified Eagle's Medium, DMEM) to form a chemically defined complete culture medium iCD1. Table 1 shows ingredients of an optimized culture medium supplement and concentrations thereof.

The present invention further provides a complete culture medium for iPS cells, which is formed by one or more of a basal culture medium, serum, and a serum replacement supplement, and the above two culture medium supplements.

The basal culture medium includes, but is not limited to, DMEM (Dulbecco's Modified Eagle's Medium), MEM (Minimal Essential Medium), BME (Basal Medium Eagle), F-10, F-12, RPMI 1640, GMEM (Glasgow's Minimal Essential Medium), αMEM (αMinimal Essential Medium), Iscove's Modified Dulbecco's Medium and M199. DMEM is preferred.

The serum replacement supplement includes, but is not limited to, KOSR, N2, B27, and ITS (Insulin-Transferrin-Selenium Supplement).

Furthermore, the culture medium supplements are configured based on the ingredients shown in Table 1, and added to a basal culture medium DMEM, to form a complete culture medium iCD1 with clear chemical ingredients. Table 1 shows ingredients of an optimized culture medium supplement and concentrations thereof. Table 2 shows ingredients of an optimized complete culture medium iCD1 and concentrations thereof.

The culture system of the present invention is a chemically defined serum-free culture system, which can be used to obtain iPS cells from somatic cells with high efficiency, maintain the growth and proliferation of the fibroblasts and the stem cells in absence of feeder cells, accelerate the iPS progression, and improve the iPS efficiency. Moreover, the culture system of the present invention is useful in eukaryotic cell culture.

**Table 1**

| Ingredients (mg/L) 25X | Final concentration(mg/L) | Supplement concentration |
|---|---|---|
| Lithium chloride | 2.12E+02 | 5.30E+03 |
| Insulin (human, recombinant) | 2.00E+01 | 5.00E+02 |
| Transferrin (human, with iron ions) | 1.00E+02 | 2.50E+03 |
| Albumin hydrolyzate (without fatty acid) | 1.00E+03 | 2.50E+04 |
| Catalase | 2.50E+00 | 6.25E+01 |
| Reduced glutathione | 1.50E+00 | 3.75E+01 |
| Superoxide dimutase (SOD) | 2.50E+00 | 6.25E+01 |
| Triiodothyronine | 2.00E-03 | 5.00E-02 |
| Adrenalone | 2.00E-02 | 5.00E-01 |
| Progesterone | 1.26E-02 | 3.15E-01 |
| Selenium | 1.60E-02 | 4.00E-01 |
| Sodium selenite | 1.04E-02 | 2.60E-01 |
| Pyruvic acid | 1.10E+02 | 2.75E+03 |
| Galactose | 1.50E+01 | 3.75E+02 |
| Putrescine | 3.22E+01 | 8.05E+02 |
| L-carnitine | 2.00E+00 | 5.00E+01 |
| Ethanolamine | 1.00E+00 | 2.50E+01 |
| Vitamin C 2-phosphate | 5.00E+01 | 1.25E+03 |
| Vitamin E | 1.00E+00 | 2.50E+01 |
| Acetylated Vitamin E | 1.00E+00 | 2.50E+01 |
| Biotin | 1.00E-01 | 2.50E+00 |
| Vitamin A | 1.00E-01 | 2.50E+00 |
| Vitamin B12 | 1.40E+00 | 3.50E+01 |
| Linoleic acid | 1.00E-01 | 2.50E+00 |
| Linolenic acid | 1.00E-01 | 2.50E+00 |
| bFGF | 5.00E-03 | 1.25E-01 |
| Glycogen synthase kinase inhibitor CHIR99021 | 1.40E+00 | 3.49E+01 |

**Table 2**

| **Inorganic ions** | **mg/L** | **Amino acids** | **mg/L** |
|---|---|---|---|
| Lithium chloride | 2.12E+02 | L-arginine | 8.40E+01 |
| Calcium chloride | 2.00E+02 | L-alanine | 8.90E+00 |
| Ferric nitrate nonahydrate | 1.00E-01 | L-asparagine | 1.32E+01 |
| Potassium chloride | 4.00E+02 | L-aspartate | 1.33E+01 |
| | | L-cysteine | 6.30E+01 |
| Magnesium sulfate | 9.77E+01 | Glycine | 4.50E+01 |
| Sodium chloride | 6.40E+03 | L-glutamine | 1.47E+01 |
| Sodium bicarbonate | 3.70E+03 | L-histidine | 4.20E+01 |
| Sodium dihydrogen phosphate monohydrate | 1.25E+02 | L-isoleucine | 1.05E+02 |
| | | L-leucine | 1.05E+02 |
| **Trace elements** | | L-lysine | 1.46E+02 |
| | | L-methionine | 3.00E+01 |
| Ammonium metavanadate | 3.00E-04 | L-phenylalanine | 6.60E+01 |
| Copper sulfate | 1.25E-03 | L-proline | 1.15E+01 |
| Manganese chloride | 5.00E-05 | L-serine | 6.30E+01 |
| Selenium | 1.60E-02 | L-threonine | 9.50E+01 |
| Sodium selenite | 1.04E-02 | L-tryptophan | 1.60E+01 |
| | | L-tyrosine | 1.04E+02 |
| **Energy substances** | | L-valine | 9.40E+01 |
| glucose | 4.50E+03 | | |
| Sodium pyruvate | 1.10E+02 | **Vitamins** | |
| Galactose | 1.50E+01 | | |
| | | Ascorbic acid | 5.00E+01 |
| **Lipids** | | Vitamin E | 1.00E+00 |
| | | Acetylated Vitamin | |
| | | E | 1.00E+00 |
| Arachidonic acid | 2.00E+00 | Biotin | 1.00E-01 |
| Cholesterol | 2.20E+02 | Pantothenic acid | 4.00E+00 |
| Linoleic acid | 1.00E+01 | Choline chloride | 4.00E+00 |
| Linolenic acid | 1.00E+01 | Folic acid | 4.00E+00 |
| Myristic acid | 1.00E+01 | Inositol | 7.20E+00 |
| Oleic acid | 1.00E+01 | Nicotinamide | 4.00E+00 |
| Palmitoleic acid | 1.00E+01 | Vitamin B6 | 4.00E+00 |
| Palmitic acid | 1.00E+01 | Riboflavin | 4.00E-01 |
| Polyether F-18 | 1.00E+05 | Thiamine | 4.00E+00 |
| Stearic acid | 1.00E+01 | Vitamin A | 1.00E-01 |
| Tween 80 | 2.20E+03 | Vitamin B12 | 1.40E+00 |

| **Growth factors/ Proteins** | | **Other substances** | |
|---|---|---|---|
| Insulin (human, recombinant) | 2.00E+01 | | |
| Transferrin (with iron ions) | 1.00E+02 | | |
| | | Putrescine | 3.22E+01 |
| Albumin hydrolyzate | 1.00E+03 | L-carnitine | 2.00E+00 |
| Catalase | 2.50E+00 | Ethanolamine | 1.00E+00 |
| Reduced glutathione | 1.50E+00 | Phenol red CHIR99021 | 1.50E+01 |
| | | | 1.40E+00 |
| Superoxide dimutase | 2.50E+00 | Mercaptoethanol | 8.17E+00 |
| Triiodothyronine | 2.00E-03 | | |
| Adrenalone | 2.00E-02 | | |
| Progesterone | 1.26E-02 | | |
| bFGF | 5.00E-03 | | |
| Mouse leukemia inhibitory factor | 1.00E-03 | | |
| (needed to be added in mouse stem cell culture) | | | |

### BRIEF DESCRIPTION OF THE DRAWINGS

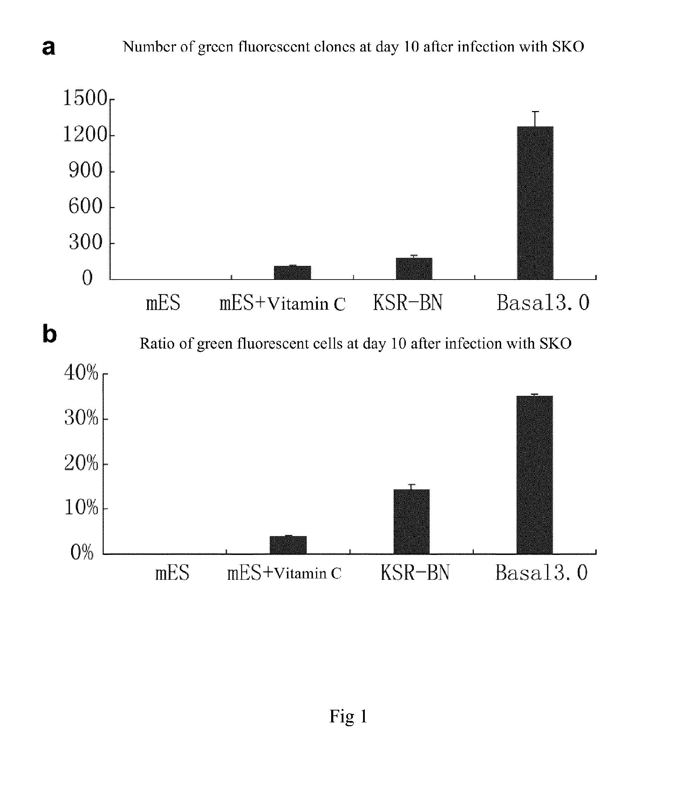

FIG. 1 shows test results of efficiency comparison of various culture schemes, in which FIG. 1a shows the number of the reprogrammed clones induced from 2 x 10⁴ embryonic fibroblasts at day 10 after infection with SKO (SOX2, KIF4, and OCT4) viruses under four culture conditions including mES, mES supplemented with vitamin C, KSR-BN, and Basal3.0 respectively (specific ingredients of the four culture conditions are described below); and FIG. 1b shows the ratio of the reprogrammed cells determined at day 10 after infection with the SKO viruses under the four culture conditions including mES, mES supplemented with vitamin C, KSR-BN, and Basal3.0 respectively.

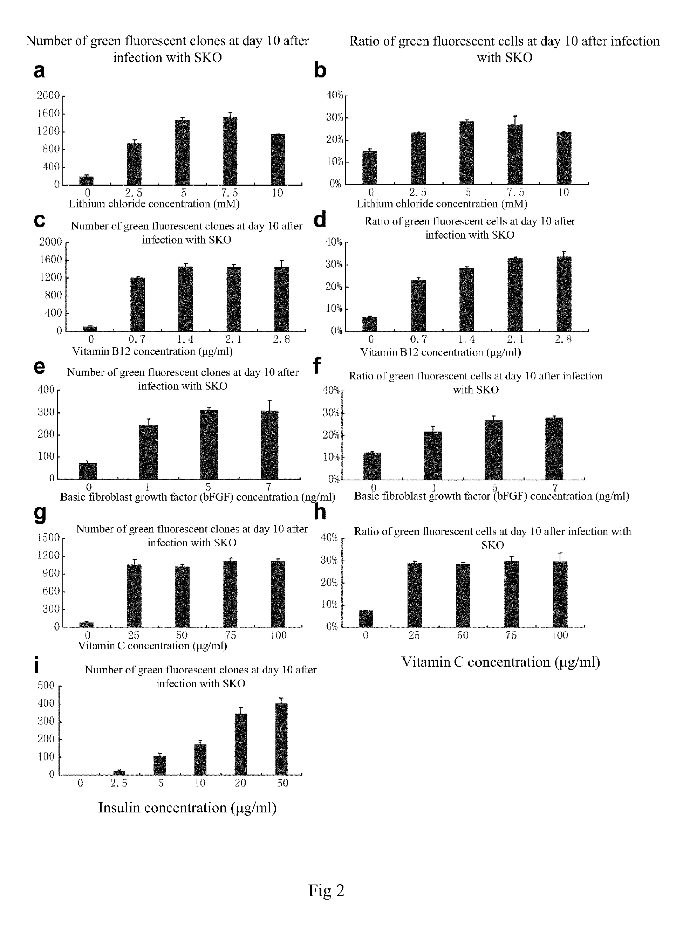

FIG. 2 shows dose-response test results of the core ingredients in the supplement, in which FIG. 2a shows a statistical result of the number of the reprogrammed clones at day 10 after infection with the SKO viruses when the concentration of lithium chloride ranges from 0 to 10 mmol/ml; FIG. 2b shows the ratio of the reprogrammed cells (green fluorescent cells) at day 10 after infection with the SKO viruses when the concentration of lithium chloride ranges from 0 to 10 mmol/ml; FIG. 2c shows a statistical result of the number of the reprogrammed clones at day 10 after infection with the SKO viruses when the concentration of vitamin B 12 ranges from 0 to 2.8 µg/ml; FIG. 2d shows the ratio of the reprogrammed cells at day 10 after infection with the SKO viruses when the concentration of vitamin B 12 ranges from 0 to 2.8 µg/ml; FIG. 2e shows a statistical result of the number of the reprogrammed clones at day 10 after infection with the SKO viruses when the concentration of bFGF ranges from 0 to 7 ng/ml; FIG. 2f shows the ratio of the reprogrammed cells at day 10 after infection with the SKO viruses when the concentration of the bFGF ranges from 0 to 7 ng/ml; FIG. 2g shows a calculation result of the number of the reprogrammed clones at day 10 after infection with the SKO viruses when the concentration of vitamin C ranges from 0 to 100 µg/ml; FIG. 2h shows the ratio of the reprogrammed cells at day 10 after infection with the SKO viruses when the concentration of vitamin C ranges from 0 to 100 µg/ml; and FIG. 2i shows a calculation result of the number of the reprogrammed clones at day 10 after infection with the SKO viruses when the concentration of insulin ranges from 0 to 50 µg/ml.

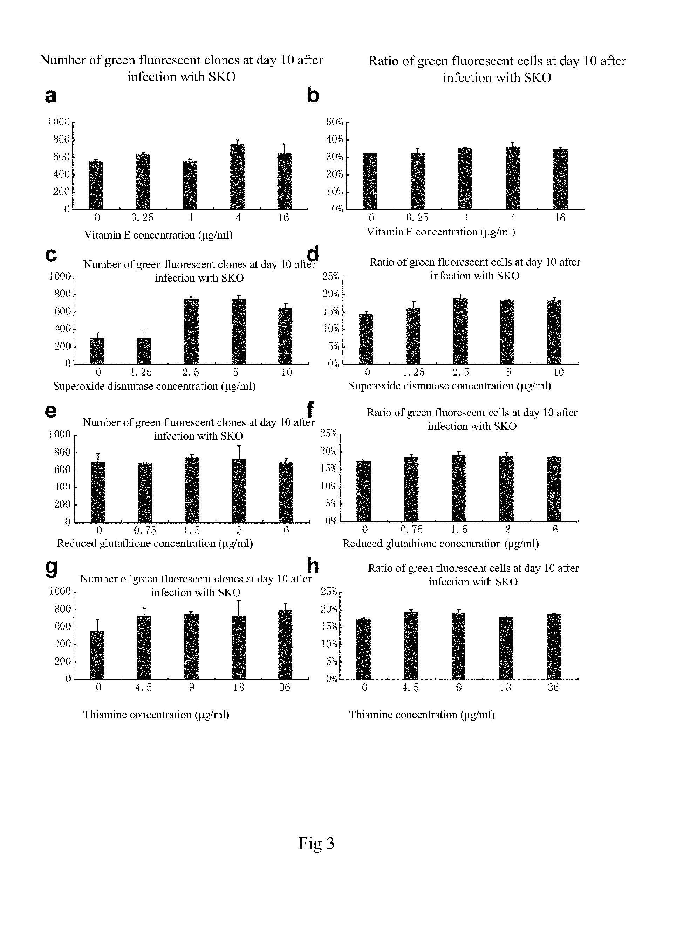

FIG. 3 shows dose-response test results of the anti-oxidants, in which FIG. 3a shows a statistical result of the number of the reprogrammed clones at day 10 after infection with the SKO viruses when the concentration of the anti-oxidant vitamin E ranges from 0 to 16 µg/ml; FIG. 3b shows the ratio of the OCT4-GFP positive cells determined at day 10 after infection by flow cytometry (FCM) when the concentration of the anti-oxidant vitamin E ranges from 0 to 16 µg/ml; FIG. 3c shows a statistical result of the number of the reprogrammed clones at day 10 after infection with the SKO viruses when the concentration of the anti-oxidant superoxide dismutase (SOD) ranges from 0 to 10 µg/ml; FIG. 3d shows the ratio of the OCT4-GFP positive cells determined at day 10 after infection by flow cytometry (FCM) when the concentration of the anti-oxidant superoxide dismutase (SOD) ranges from 0 to 10 µg/ml; FIG. 3e shows a statistical result of the number of the reprogrammed clones at day 10 after infection with the SKO viruses when the anti-oxidant reduced glutathione ranges from 0 to 6 µg/ml; FIG. 3f shows the ratio of the OCT4-GFP positive cells determined at day 10 after infection by flow cytometry (FCM) when the concentration of the anti-oxidant reduced glutathione ranges from 0 to 6 µg/ml; FIG. 3g shows a statistical result of the number of the reprogrammed clones at day 10 after infection with the SKO viruses when the concentration of the anti-oxidant thiamine ranges from 0 to 36 µg/ml; and FIG. 3h shows the ratio of the OCT4-GFP positive cells determined at day 10 after infection by flow cytometry (FCM) when the concentration of Thiamine ranges from 0 to 36 µg/ml.

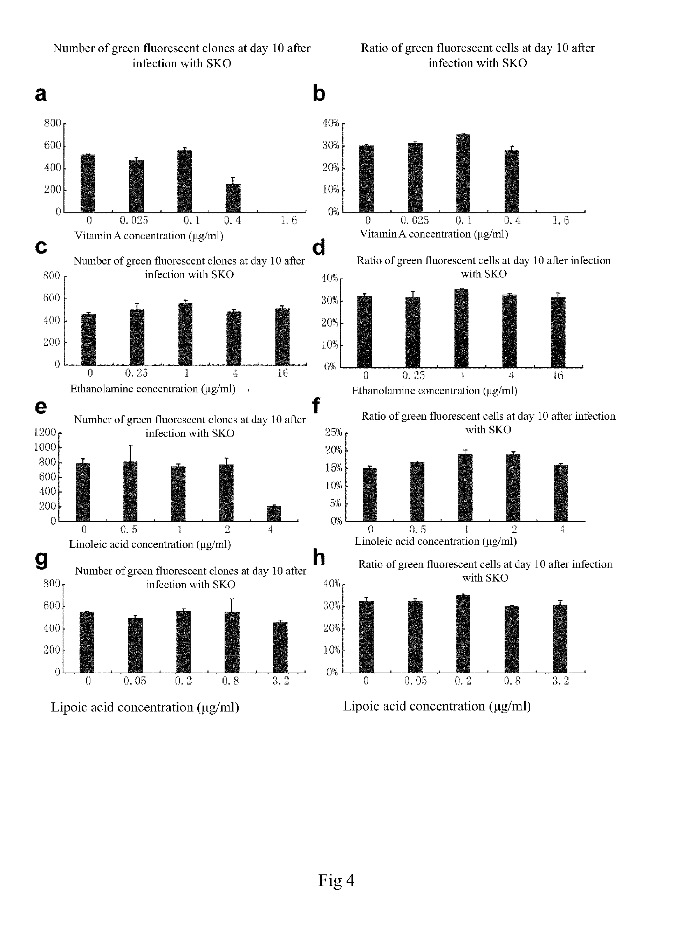

FIG. 4 is dose-response graphs of the growth supporting substances, in which FIG. 4a shows a statistical result of the number of the reprogrammed clones at day 10 after infection with the SKO viruses when the concentration of the growth supporting substance vitamin A ranges from 0 to 1.6 µg/ml, FIG. 4b shows the ratio of the OCT4-GFP positive cells determined at day 10 after infection by flow cytometry (FCM) when the concentration of the growth supporting substance vitamin A ranges from 0 to 1.6 µg/ml; FIG. 4c shows a statistical result of the number of the reprogrammed clones at day 10 after infection with the SKO viruses when the concentration of the growth supporting substance ethanolamine ranges from 0 to 16 µg/ml; FIG. 4d shows the ratio of the OCT4-GFP positive cells determined at day 10 after infection by flow cytometry (FCM) when the concentration of the growth supporting substance ethanolamine ranges from 0 to 16 µg/ml; FIG. 4e shows a statistical result of the number of the reprogrammed clones at day 10 after infection with the SKO viruses when the concentration of the growth supporting substance linoleic acid ranges from 0 to 4 µg/ml; FIG. 4f shows the ratio of the OCT4-GFP positive cells determined at day 10 after infection by flow cytometry (FCM) when the concentration of the growth supporting substance linoleic acid ranges from 0 to 4 µg/ml; FIG. 4g shows a statistical result of the number of the reprogrammed clones at day 10 after infection with the SKO viruses when the concentration of lipoic acid ranges from 0 to 3.2 µg/ml; and FIG. 4h shows the ratio of the OCT4-GFP positive cells determined at day 10 after infection by flow cytometry (FCM) when the concentration of the growth supporting substance linoleic acid ranges from 0 to 4 µg/ml.

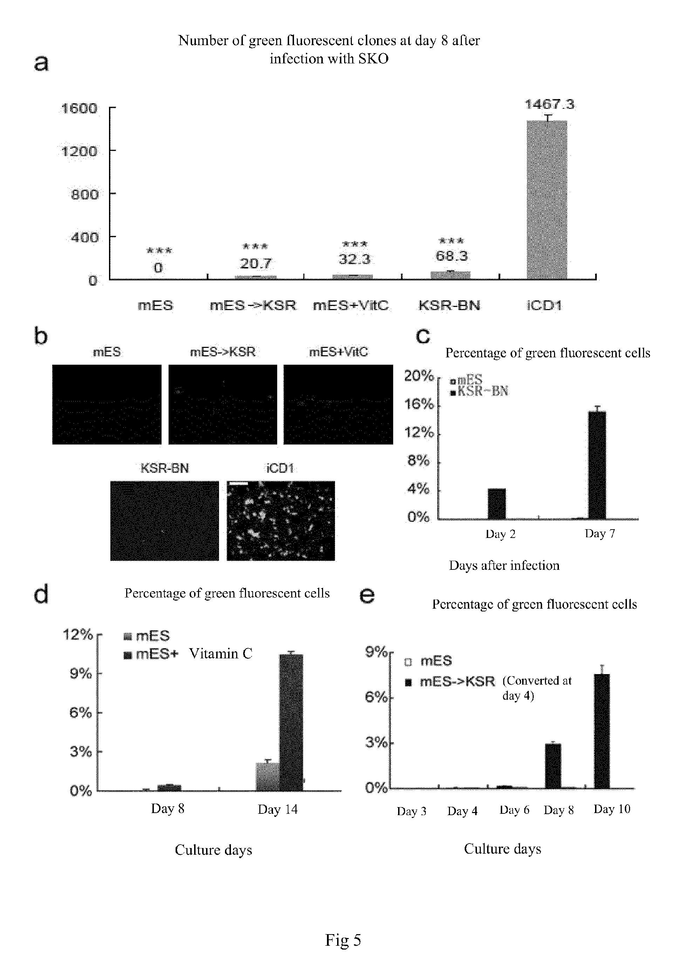

FIG. 5 compares effects of the culture medium formulation iCD1 (Basal3.0 is supplemented with glycogen synthase kinase-3 inhibitor CHIR99021) according to the present invention and the reported advanced induced reprogramming culture schemes, in which FIG. 5a compares the efficiencies of the chemically defined culture medium formulation iCD1 according to the present invention and the reported reprogramming culture schemes; FIG. 5b shows the number of the reprogrammed clones at day 8 after infection with the several different culture schemes shown in FIG. 5a; FIG. 5c shows that the KSR-BN culture scheme improves the reprogramming efficiency (the ingredients are described below); FIG. 5d shows that the mES improves the reprogramming efficiency after being supplemented with vitamin C; and FIG. 5e shows that the mES→KSR culture scheme improves the reprogramming efficiency (a specific process of the mES->KSR culture scheme is described below).

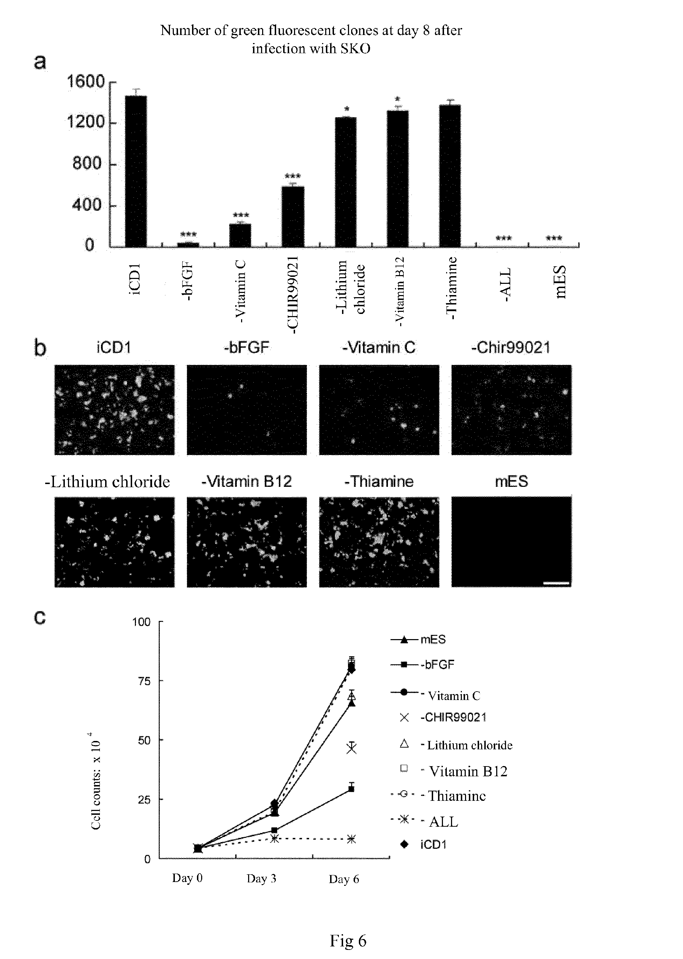

FIG. 6 shows the effects of the six ingredients, bFGF, vitamin C, CHIR99021, lithium chloride, vitamin B12, and thiamine in the culture medium supplement according to the present invention on the reprogramming efficiency and growth, in which FIG. 6a shows the effects of the six ingredients on the reprogramming efficiency according to the present invention; FIG. 6b shows reprogramming images at day 8 after infection under the different culture conditions shown in FIG. 6a; and FIG. 6c shows growth curves under these culture conditions.

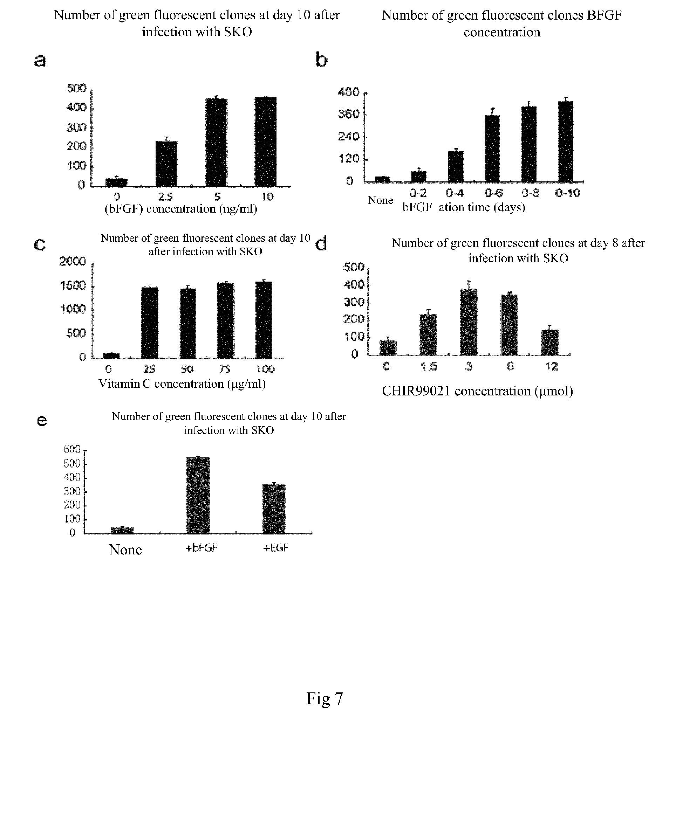

FIG. 7 shows dose responses of bFGF, vitamin C, and CHIR99021, action time of bFGF, and test results of other receptor tyrosine kinases, in which FIG. 7a shows dose response of bFGF; FIG. 7b shows test of the action time of bFGF; FIG. 7c shows dose response of vitamin C; FIG. 7d shows dose response of CHIR99021; and FIG. 7d shows effect of various receptor tyrosine kinases on reprogramming.

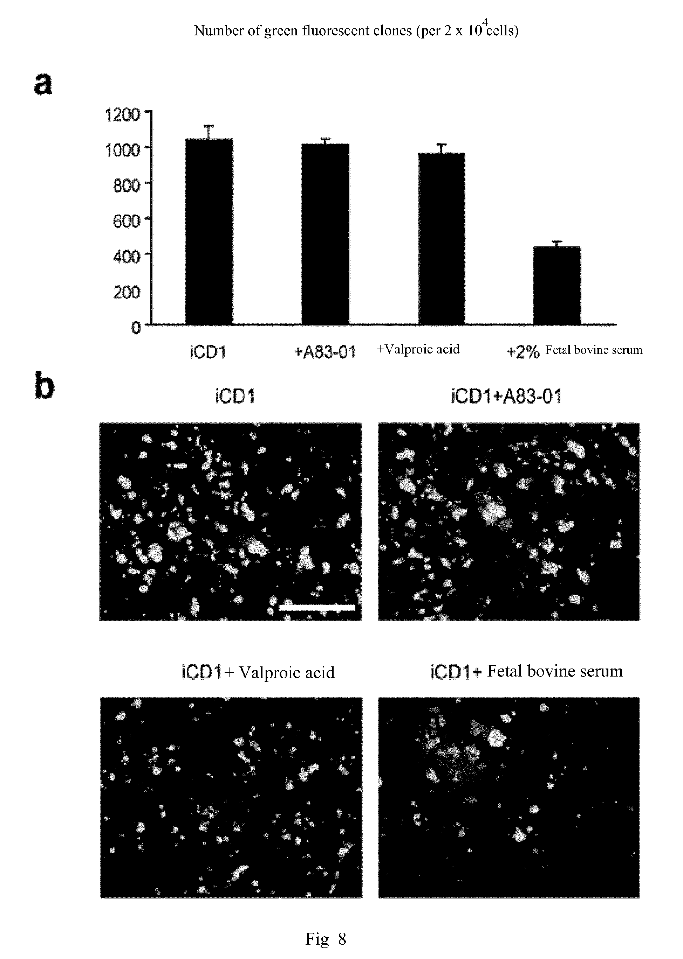

FIG. 8 shows test results that in the chemically defined culture medium iCD1, an ALK5 (a receptor of tumor necrosis factor TNF-beta) inhibitor A83-01, a histone methylase inhibitor valproic acid (VPA), and fetal bovine serum cannot further improve the reprogramming efficiency mediated by OKS, in which FIG. 8a shows the number of the reprogrammed clones calculated at day 10 after the mouse fibroblasts are infected with SKO viruses, and then cultured respectively in the chemically defined culture medium iCD1, and iCD1 culture media supplemented with A83-01 (0.5 µM), valproic acid (1 mM), and 2% fetal bovine serum; and FIG. 8b shows typical reprogrammed clones in the tests shown in FIG. 8a.

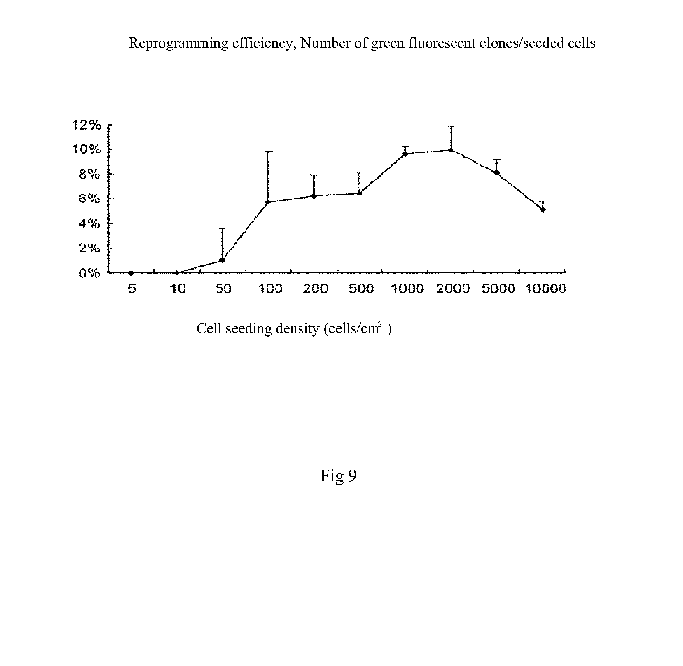

FIG. 9 shows a test result of the effect of the initial cell seeding density on the reprogramming efficiency.

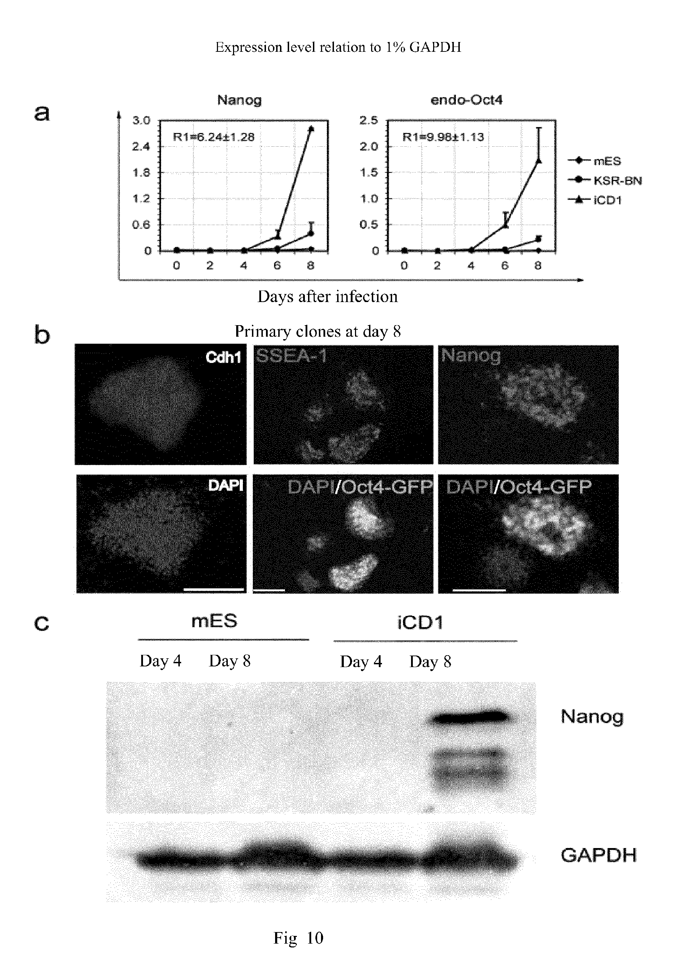

FIG. 10 shows expression results of the endogenous pluripotent molecular markers under an iCD1 culture condition, in which FIG. 10a shows expressions of the endogenous pluripotent molecular markers detected by real-time fluorescent quantitative PCR; FIG. 10b shows results of immunofluorescence assays performed by directing fixing on a cell culture plate; and FIG. 10c shows expression profiles detected by immunoblotting.

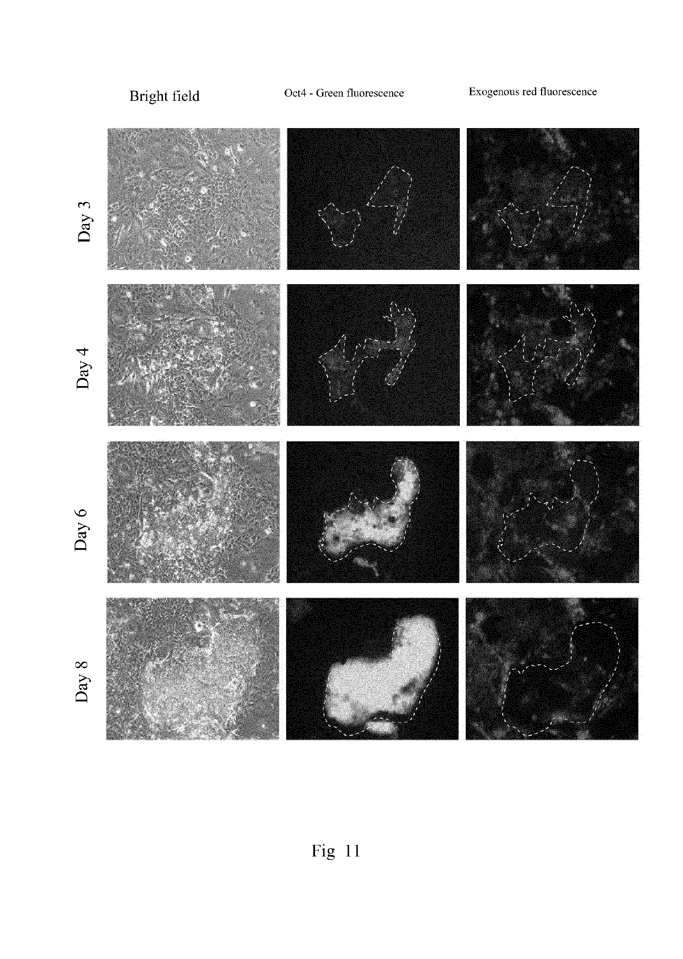

FIG. 11 shows reprogramming tracked in the iCD1 culture system.

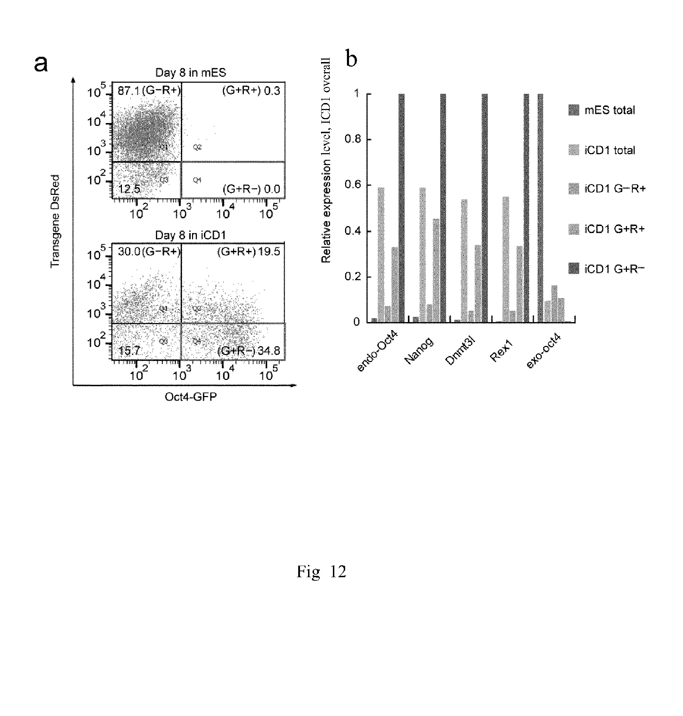

FIG. 12 shows a reprogramming efficiency at day 8 after infection, and expression profiles of the related molecular markers, in which FIG. 12a shows the ratio of the reprogrammed cells (green fluorescent cells) to non-reprogrammed cells (red fluorescent cells) detected at day 8 after the Oct4-GFP mouse embryonic fibroblasts (MEFs) are infected with SKO viruses and exogenous red fluorescent protein DsRed viruses, and then cultured respectively in mES and iCD1 culture media; and FIG. 12b shows expression profiles of the pluripotent molecular markers, detected by real-time fluorescent quantitative PCR, in different cell populations infected with SKO viruses and then cultured respectively in mES and iCD1 for 8 days, and the populations sorted from 8-day culture in iCD1.

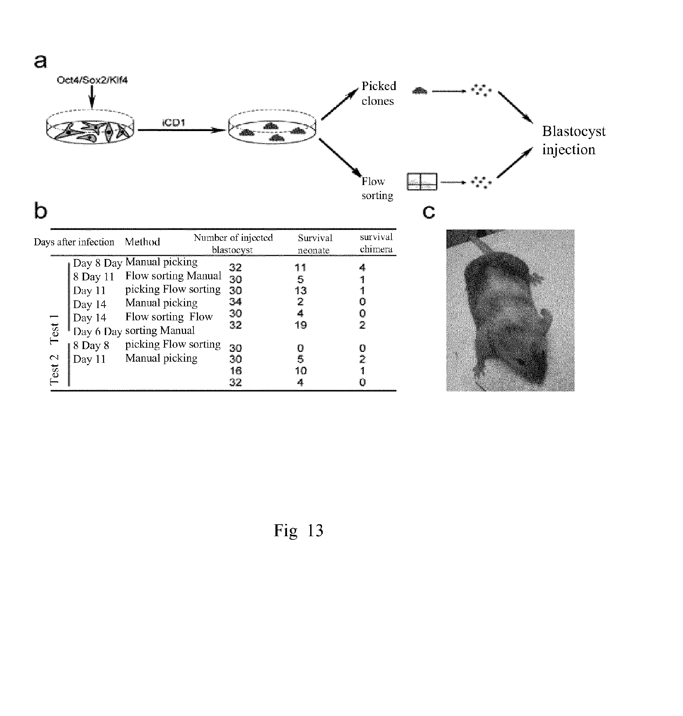

FIG. 13 shows related test results of the chimeric mice construction process, in which FIG. 13a shows pluripotency of iPS cells induced by SKO viruses in iCD1 culture medium, as detected by embryo injection; FIG. 13b shows a statistical result of the test shown in FIG. 13a implemented at different induction days; FIG. 13c shows chimeric mouse generated by injecting iPS cells obtained at day 8 into a blastocyst; and the test suggests that after being reprogrammed in iCD1 for 8 days, the cells infected with SKO viruses can form pluripotent stem cells able to generate a chimera.

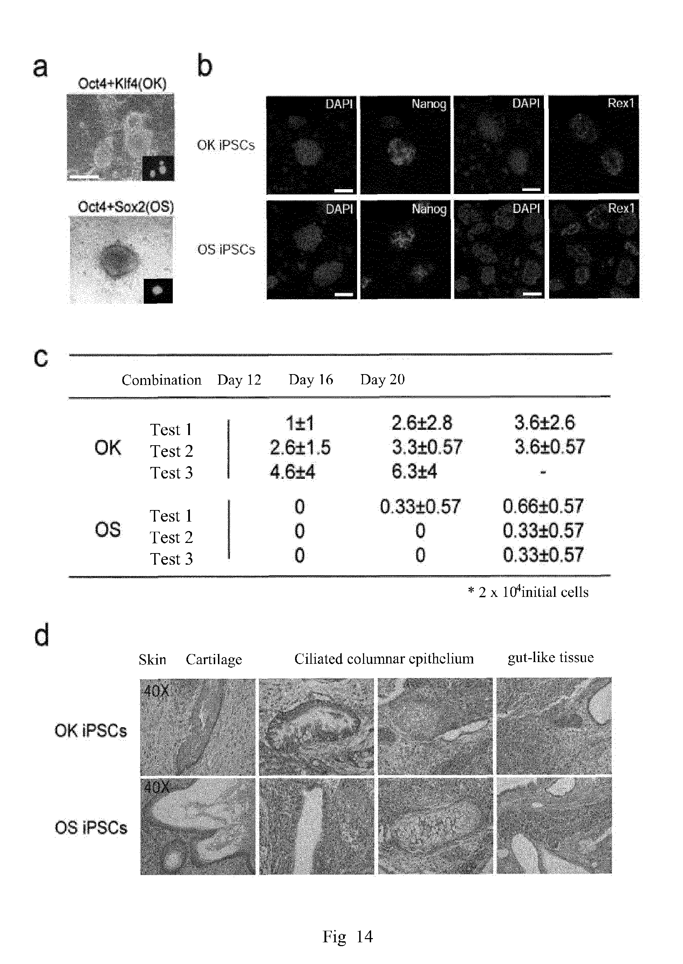

FIG. 14 shows test results of the somatic cells reprogrammed with OK and OS in the iCD1 culture system; in which FIG. 14a shows primary clone picture and passage picture of the somatic cells reprogrammed with OK and OS under iCD1 culture condition; FIG. 14b shows expression profiles of the stem cell specific molecular markers found through immunofluorescence detection; FIG. 14c shows implementation of reprogramming tests with OK and OS in the iCD1 culture system, and statistical efficiencies thereof; and FIG. 14d shows teratoma sections, suggesting that the pluripotent stem cells formed by reprogramming with OK and OS have the characteristics of embryonic stem cells.

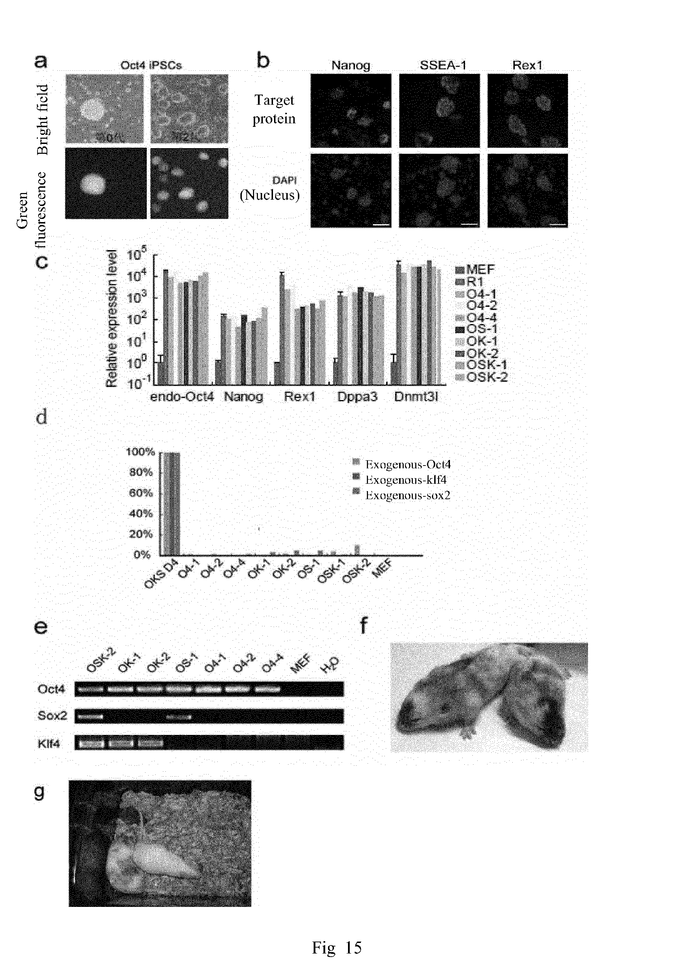

FIG. 15 shows related test results of the MEFs reprogrammed with OCT4 under iCD1 condition, in which FIG. 15a shows morphological pictures of primary clones induced with Oct4 at day 33 after infection and the formed cell lines; FIG. 15b shows expressions of the pluripotent molecular markers by Oct4 iPS clones; FIG. 15c shows that the picked clones all express stem cell specific pluripotent molecular markers as detected by real-time fluorescent quantitative PCR; FIG. 15d shows expression profile of the exogenous gene analyzed by quantitative RT-PCR; FIG. 15e shows that the picked clones are induced by a single factor Oct4, instead of contamination, as determined and analyzed by insertion assay; FIG. 15f shows chimeric mice generated by injecting Oct4 iPSC cells into a blastocyst; and FIG. 15g shows that the generated chimeric mice have germline mosaic competence.

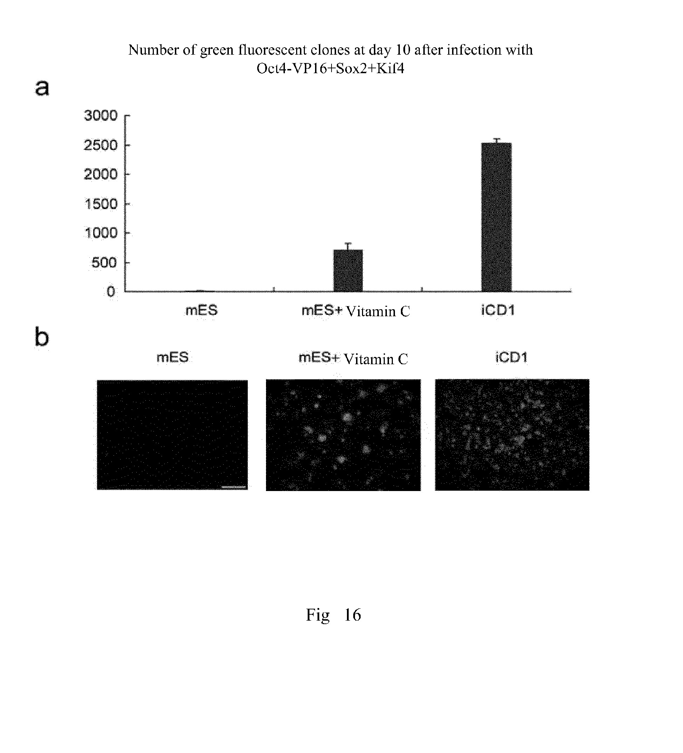

FIG. 16 shows related test that iCD1 can improve the reprogramming efficiency, in case of infection with Oct4-VP16 (VP16 is a gene sequence derived from viruses, the linking of VP16 to a transcription factor can enhance the activity of the transcription factor), Klf4 and Sox2, in which FIG. 16a shows the number of the fluorescent clones calculated at day 10 after the Oct4-GFP MEFs are infected with Oct4-vp16SK, and cultured respectively in the mES culture medium, mES culture medium supplemented with vitamin C, and iCD1 culture medium; and FIG. 16b shows typical pictures taken in the test as shown in FIG. 16a.

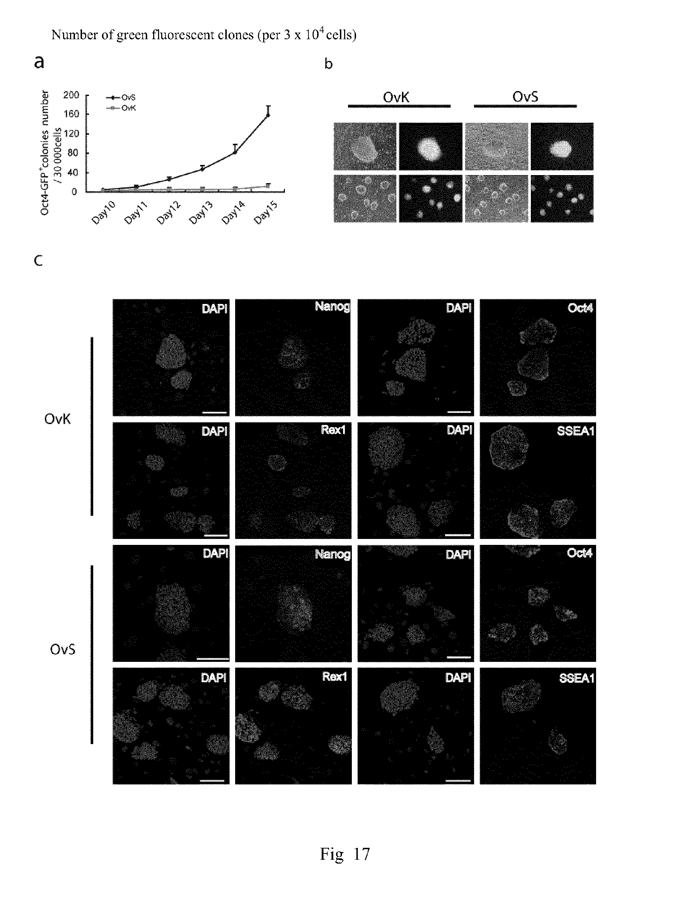

FIG. 17 shows related tests that Oct4-vp16/Kif4 and Oct4-vp16/Sox2 reprogram somatic cells in the iCD1 culture system, in which FIG. 17a shows the number of the reprogrammed clones calculated respectively at different days after the Oct4-GFP MEFs are infected respectively with Oct4-vp16/Kif4 and Oct4-vp16/Sox2, and cultured in the iCD1 system; FIG. 17b shows typical pictures of iPS clones induced with Oct4-vp16K and Oct4-vp16S; and FIG. 17c shows expressions of typical pluripotent molecular markers by iPS cells induced with Oct4-vp16K and Oct4-vp16S.

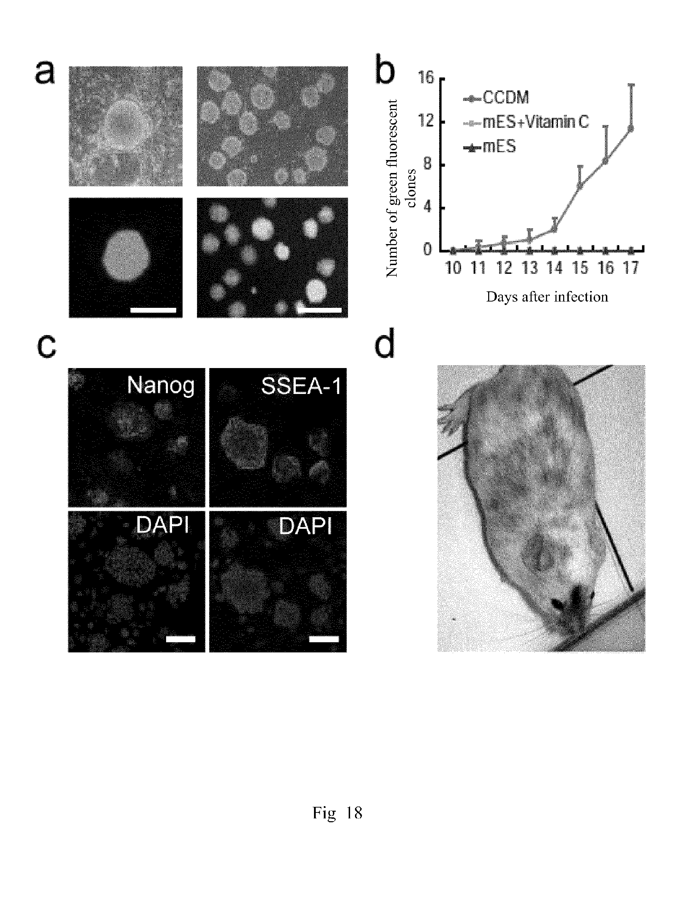

FIG. 18 shows related tests that Oct4-vp16 reprograms somatic cells in the iCD1 culture system, in which FIG. 18a shows typical pictures of iPS clones induced with Oct4-vp16; FIG. 18b shows the number of the reprogrammed clones calculated at different days after Oct4-GFP MEFs are infected with Oct4-vp16, and then cultured respectively in mES, mES supplemented with vitamin C, and iCD1 culture medium; FIG. 18c shows the expression of the pluripotent molecular markers by the clones induced with Oct4-vp16; and FIG. 18d shows a picture of chimeric mouse generated by injecting Oct4-vp16 induced iPS clones into a blastocyst.

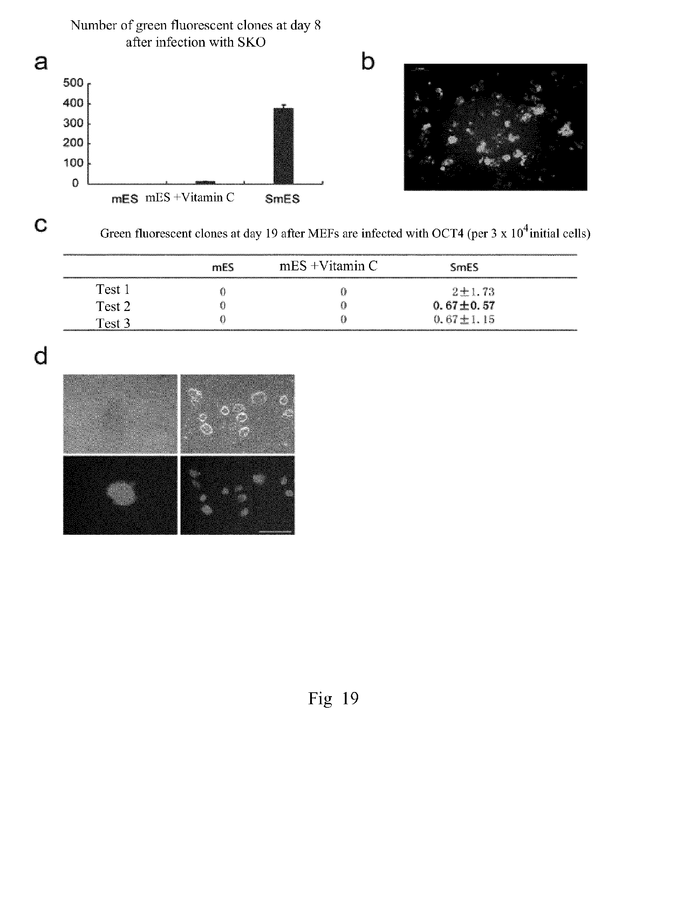

FIG. 19 shows tests of reprogramming facilitated by supplementing iCD1 core ingredients into the mES culture medium, in which FIG. 19a shows the number of the reprogrammed clones calculated at day 8 after the Oct4-GFP MEFs are infected with SKO viruses, and then cultured respectively in mES, mES + vitamin C, SmES (mES supplemented with vitamin C, bFGF, CHIR99021, lithium chloride, and B27) culture media; FIG. 19b shows a typical fluorescent clone picture at day 8 under SmES culture condition; FIG. 19c shows the number of the fluorescent clones observed at different days after Oct4-GFP MEFs are infected with OCT4 viruses, and then cultured respectively in mES, mES + vitamin C, and iCD1 culture media; and FIG. 19d shows primary clone picture and passage clone picture of the somatic cells reprogrammed with OCT4 under SmES culture system.

### DETAILED DESCRIPTION OF THE INVENTION

In order to make the present invention more comprehensible, the present invention is further described with reference to specific examples. It should be understood that these examples are only used to illustrate the present invention, but not limit the scope thereof.

It should be noted that in the examples below, unless specifically stated otherwise, the terms and ingredients of the culture medium are as described below.

The basal culture medium is an artificially prepared culture containing carbohydrates, amino acids, inorganic salts, vitamins, lipids, and other nutrients required for cell growth. The basal culture medium in the present invention includes, but is not limited to, Dulbecco's Modified Eagle's Medium (DMEM), Minimal Essential Medium (MEM), Basal Medium Eagle (BME), F-10, F-12, RPMI 1640, Glasgow's Minimal Essential Medium (GMEM), αMinimal Essential Medium (αMEM), Iscove's Modified Dulbecco's Medium, and M199, with the preferred basal culture medium being high sugar DMEM basal culture medium.

The fetal bovine serum is a culture supporting cell growth isolated and extracted from fetal bovine blood, which contains abundant nutrients and growth factors and can support the growth of a variety of types of cells, but the specific ingredients of which are unknown. Therefore, the fetal bovine serum has the disadvantages such as unclear ingredients, high batch difference, and potential presence of animal pathogens.

KOSR is a class of commercialized culture supplements replacing serum, and generally has clear ingredients. The KOSR described herein includes, but is not limited to, serum replacement supplements such as KSR (a commercialized serum-containing culture supplement), N2 (a commercialized serum replacement supplement, and ingredients thereof include insulin, transferrin, progesterone, putrescine, and sodium selenite), and B27 (a serum free culture supplement used to culture nerve cells).

Embryonic stem cells are shortly referred to as ES or EK cells. The ES cells are a class of cells isolated from an early embryo (before gastrula stage) or from primordial gonad, which have properties of unlimited proliferation in in-vitro culture, self-renewal, and multipotent differentiation. In-vitro culture of the mouse ES cells requires the support of the feeder cells and the mouse leukemia inhibitory factor (LIF) to maintain the pluripotent state. It is found through researches that in an in-vitro culture process, use of a glycogen synthase kinase inhibitor and a mitogen activated protein kinase inhibitor in combination can inhibit the differentiation of the ES cells, and maintain the pluripotent state of the ES cells in absence of feeder cells and mouse LIF.

Glycogen synthase kinase-3 (GSK-3), which is a multifunctional serine/threonine protein kinase, is an important component in multiple intracellular signaling pathways, and is involved in a variety of physiological processes such as intracellular glucose metabolism, cell proliferation, and cell differentiation and apoptosis. The GSK inhibitor mainly includes, for example, CHIR99021, BIO, and SB216763. CHIR99021 is preferred, and the structure thereof is:

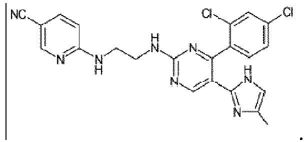

iPS cells are an abbreviation for induced pluripotent stem cells, and are a pluripotent stem cell state restored by treating differentiated cells with reprogramming factors. At present, it is confirmed through a tetraploid blastocyst injection technology that the iPS cells can produce a complete mouse, suggesting that the iPS cells are pluripotent.

The reprogramming factors refer to factors able to restore differentiated cells to a pluripotent state, and are mostly nuclear transcription factors. The currently reported transcription factors for reprogramming include, for example, SOX2, OCT3, KIF4, NONOG, LIN28, c-myc, lin28, esrrb, and tbx3. The transcription factors as described herein include, but are not limited to, the above transcription factors.

The ingredients of a feeder medium include a high-sugar basal culture medium (DMEM) supplemented with 10% fetal bovine serum (FBS) (Hyclone), 2 mM glutamine, and non-essential amino acids (NEAAs).

The ingredients of a serum-containing ES cell culture medium include a high-sugar basal culture medium (DMEM, a basal culture medium named with a first letter of the inventor), 15% fetal bovine serum FBS (GIBCO), 2 mM glutamine, non-essential amino acids (NEAAs), penicillin/streptomycin, beta-mercaptoethanol, and sodium pyruvate.

mES is a classical stem cell culture medium, and has the following ingredients: high-sugar DMEM culture medium supplemented with 15% fetal bovine serum, non-essential amino acids, glutamine, penicillin/streptomycin, beta-mercaptoethanol, pyruvic acid, and leukocyte inhibitory factor (a growth factor maintaining the pluripotent state of mice).

KSR serum-free culture medium: KSR is an abbreviation for Knockout Serum Replace, and is a commercialized serum replacement stem cell culture supplement. The KSR complete culture medium used in examples of the present invention includes two types, one is used in an induction process of the iPS cells, and ingredients thereof include a high-sugar basal culture medium (DMEM), 10% KSR supplement, 2 mM glutamine, non-essential amino acids (NEAAs), penicillin/streptomycin, beta-mercaptoethanol, and sodium pyruvate. The other one is a complete KSR serum-free culture medium used to culture stem cells or iPS clones, and ingredients thereof include KNOCKOUT DMED (a basal culture medium with optimized osmotic pressure and suitable for stem cell culture), 15% KSR supplement, 2 mM glutamine, non-essential amino acids (NEAAs), penicillin/streptomycin, and Beta-mercaptoethanol. All culture media for iPS process and clone are supplemented with mouse leukocyte inhibitory factor LIF (millipore, trade name ESGRO, a growth factor inhibiting differentiation of mouse stem cells at a finally concentration of 1000 U/ml.

KSR-BN is the KSR complete culture medium used in the induction process of the iPS cells and supplemented with bFGF and N2 (a commercialized serum replacement supplement having ingredients including insulin, transferrin, progesterone, putrescine, and sodium selenite).

Basal3.0 complete culture medium refers to one of the culture media described in the present invention, and is specifically formed by a high-sugar basal culture medium (DMEM) supplemented with vitamin C, insulin, lithium chloride, vitamin B 12, an anti-oxidant, a receptor tyrosine kinase growth factor, and a series of growth supporting substances. The specific ingredients are shown in Table 2, in which CHIR99021 is absent.

iCD1 complete culture medium is one of the culture media described in the present invention, and is formed by mixing the supplements as shown in FIG. 1 with a high-sugar basal culture medium (DMEM), glutamine, non-essential amino acids, penicillin/streptomycin, beta-mercaptoethanol, and sodium pyruvate, in which a ratio by volume of the supplement in Table 1 to other ingredients is 1:24. The specific ingredients and concentrations thereof are shown in Table 2.

Furthermore, unless specifically stated otherwise, somatic cells reprogramming based on mice are carried out by employing the following process.

Somatic cell type used in reprogramming is OG2 MEFs of no more than 3 passages. OG2 mice are transgenic mice with green fluorescent protein (GFP) gene linked after the promoter of Oct4 gene specifically expressed by stem cell. At a later stage of the reprogramming, endogenous Oct4 of OG2 MEF is activated, and the green fluorescent protein is concomitantly expressed, so the cells or reprogrammed clones appear green when observed under a fluorescence microscope. The reprogramming efficiencies under different conditions can be compared by a research by directly counting the reprogrammed clones, that is, the green fluorescent clones under a fluorescence microscope, or by analyzing the ratio of the green fluorescent cells through flow cytometry (FCM).

Cell assay and preparation of viruses are as follows. Cells are seeded at a density of 2 x 10⁴ cells/well in a 12-well attached cell culture plate. 6-18 hrs after the cell seeding, the cells are infected with the viruses carrying a mouse reprogramming factor according to the cell density and state. The infection is carried out in two rounds, 24 hrs after the first round of infection, the second round of infection is carried out, and the virus fluid is replaced by various test media 24 hrs after the second round of infection. The day of replacing the virus fluid is recorded as day 0 (D0), and the GFP fluorescent clones are counted in the original wells or the ratio of the GFP fluorescent cells are analyzed by flow cytometry (FCM) at different times after infection as desired.

The preparation of virus includes transfecting virus packaging cells (PlatE) with reprogramming factor plasmid cloned onto a PMX vector, refreshing the culture media 12-16 hrs after infection, using the culture collected 48 hrs after the transfection as a virus fluid for the first infection, and using the additional culture collected 24 hrs after adding fresh culture as a virus fluid for the second infection.

In addition, unless specifically stated otherwise, some abbreviations should be understood as follows.

SKO specifically refers to SOX2, KIF4, and OCT4 viruses, or to SOX2, KIF4, and OCT4 virus-infected cells, and has the same meaning as three factors. OK specifically refers to OCT4 and KIF4 viruses, or to OCT4 and KIF4 virus-infected cells, and OS specifically refers to OCT4 and SOX2 viruses, or to OCT4 and SOX2 virus-infected cells.

### Example 1: Comparison of reprogramming efficiencies of B3.0 and other culture schemes, and ingredient concentration optimization

SKO viruses were mixed at 1:1:1 (0.5 ml each), and then 2 x 10⁴ fibroblasts in total in a well of a 12-well plate were infected, and cultured at 37°C under 5% CO₂ respectively in mES culture medium, mES supplemented with vitamin C, KSR-BN culture medium, and Basal 3.0 culture medium. At day 10 after infection, the reprogrammed clones were directly counted under a fluorescence microscope, and the ratio of the reprogrammed cells was analyzed through flow cytometry. FIG. 1a shows the number of the reprogrammed clones induced from 2 x 10⁴ Oct4-GFP transgenic embryonic fibroblasts at day 10 after infection under four culture conditions including mES, mES supplemented with vitamin C, KSR-BN, and Basal3.0, in which the replications n in each group of test are 3, and the error bar represents standard deviation (SD). The result suggests that at day 10 after infection, 1278 reprogrammed clones appear under the Basal3.0 culture condition, while only 180 and 113 reprogrammed clones respectively appear under the culture conditions of KSR-BN and mES supplemented with vitamin C, and almost no reprogrammed clones appear under the mES culture condition. FIG. 1b shows the ratio of the OCT4-GFP positive cells determined at day 10 after infection by flow cytometry (FCM) under four culture conditions including mES, mES supplemented with vitamin C, KSR-BN, and Basal3.0, in which the replications n in the test are 3, and the error bar represents standard deviation (SD). The result suggests that at day 10 after infection, 35.2% of cells are reprogrammed in the Basal3.0 culture system, while only 14.25% and 3.9% of cells are respectively reprogrammed under the conditions of KSR-BN and mES supplemented with vitamin C. The two test results indicate that Basal3.0 is a very excellent culture system for inducing reprogramming with three factors, OCT4, Sox2, and Kif4.

In order to determine the effects of the ingredients in Basal3.0 on reprogramming under three-factor infection conditions and the optimum working concentration ranges thereof, different concentrations of different ingredients in Basal3.0 are used in iPS efficiency test. Both reprogrammed clone number and reprogrammed cell ratio are used as evaluation indexes, so as to accurately determine the effects of the test substances on the reprogramming. FIG. 2 shows dose-response test results of core ingredients. FIG. 2a shows the number of the reprogrammed clones formed by inducing with Basal3.0 at day 10 after infection when the dosage range of lithium chloride is from 0 to 10 mmol/ml; and FIG. 2b shows the ratio of the reprogrammed cells determined under the corresponding test condition. FIG. 2c shows the number of the reprogrammed clones formed by inducing with Basal3.0 at day 10 after infection when the dosage range of vitamin B 12 is from 0 to 2.8 mg/ml, and FIG. 2d shows the ratio of the reprogrammed cells determined under the corresponding test condition. FIG. 2e shows the number of the reprogrammed clones formed by inducing with Basal3.0 at day 10 after infection when the dosage range of bFGF is from 0 to 7 ng/ml, and FIG. 2f shows the ratio of the reprogrammed cells determined under the corresponding test condition. FIG. 2g shows the number of the reprogrammed clones formed by inducing with Basal3.0 at day 10 after infection when the dosage range of vitamin C is from 0 to 100 mg/ml, and FIG. 2h shows the ratio of the reprogrammed cells determined under the corresponding test condition. FIG. 2i shows the number of the reprogrammed clones formed by inducing with Basal3.0 at day 10 after infection when the dosage range of insulin is from 0 to 50 mg/ml. In the above tests, the replications n in each group of test are 3, and the error bar represents standard deviation (SD). This series of substances on reprogramming are significant in the tests, and thus are considered as core substance affecting reprogramming in Basal3.0, and recommended working concentrations of lithium chloride, vitamin B 12, bFGF, vitamin C, and insulin are respectively, but not limited to, 5 mmol/ml, 1.4 µg/ml, 5 ng/ml, 50 µg/ml, and 50 µg/ml.

FIG. 3 shows dose-response test results of a series of anti-oxidants. FIG. 3a shows the number of the reprogrammed clones formed by inducing with Basal3.0 at day 10 after infection when the concentration of the anti-oxidant vitamin E ranges from 0 to 16 µg/ml, and FIG. 3b shows the ratio of the reprogrammed cells determined under the corresponding test condition. FIG. 3c shows the number of the reprogrammed clones formed by inducing with Basal3.0 at day 10 after infection when the concentration of the anti-oxidant superoxide dismutase (SOD) ranges from 0 to 10 µg/ml, and FIG. 3d shows the ratio of the reprogrammed cells determined under corresponding test condition. FIG. 3e shows the number of the reprogrammed clones formed by inducing with Basal3.0 at day 10 after infection when the concentration of the anti-oxidant reduced glutathione ranges from 0 to 6 µg/ml, and FIG. 3f shows the ratio of the reprogrammed cells determined under the corresponding test condition. FIG. 3g shows the number of the reprogrammed clones formed by inducing with Basal3.0 at day 10 after infection when the concentration of the anti-oxidant thiamine ranges from 0 to 36 µg/ml, and FIG. 3h shows the ratio of the reprogrammed cells determined under the corresponding test condition. In the above tests, the replications n in each group of test are 3, and the error bar represents standard deviation (SD). Recommended working concentrations of vitamin E, superoxide dimutase (SOD), reduced glutathione, and thiamine are respectively 1 mg/ml, 2.5 mg/ml, 1.5 mg/ml, and 9 mg/ml.

FIG. 4 shows dose-response test results of some other growth supporting substances. FIG. 4a shows the number of the reprogrammed clones formed by inducing with Basal3.0 at day 10 after infection when the concentration of vitamin A ranges from 0 to 1.6 µg/ml, and FIG. 4b shows the ratio of the reprogrammed cells determined under the corresponding test condition. FIG. 4c shows the number of the reprogrammed clones formed by inducing with Basal3.0 at day 10 after infection when the concentration of ethanolamine ranges from 0 to 16 µg/ml, and FIG. 4d shows the ratio of the reprogrammed cells determined under the corresponding test condition. FIG. 4e shows the number of the reprogrammed clones formed by inducing with Basal3.0 at day 10 after infection when the concentration of linoleic acid ranges from 0 to 4 µg/ml, and FIG. 4f shows the ratio of the reprogrammed cells determined under the corresponding test condition. FIG. 4g shows the number of the reprogrammed clones formed by inducing with Basal3.0 at day 10 after infection when the concentration of lipoic acid ranges from 0 to 3.2 µg/ml, and FIG. 4h shows the ratio of the reprogrammed cells determined under the corresponding test condition. In the above tests, the replications n in each group of test are 3, and the error bar represents standard deviation (SD). Recommended working concentrations of vitamin A, ethanolamine, linoleic acid, and lipoic acid are respectively 0.1 µg/ml, 1 µg/ml, 1 µg/ml, and 0.2 µg/ml.

### Example 2: Modification of Basal3.0

In concentration test of the ingredients in Basal3.0, the high effect of lithium chloride on reprogramming implicates that inhibition of the glycogen synthase kinase may be a factor promoting reprogramming. Therefore, a series of glycogen synthase kinase inhibitors including CHIR99021, BIO, and SB31xxxx are selected as candidates for the iPS test (two indexes the number of the reprogrammed clones and the ratio of the reprogrammed cells are used as evaluation indexes of reprogramming efficiency). As a result, the GSK3-β inhibitor CHIR99021 can significantly improve the reprogramming efficiency of Basal3.0, and thus the modified Basal3.0, that is, the chemically defined culture medium supplemented with (without limitation) the glycogen synthase kinase-3 (GSK3-β) inhibitor CHIR99021 is named as iCD1.

Through the iPS efficiency test, reprogramming efficiencies of iCD1 and all advanced culture schemes currently reported are compared, in which the advanced culture schemes include mES supplemented with vitamin C, mES to KSR (that is, at day 4 after infection, a classical embryonic stem cell culture medium (mES) is converted to a commercialized serum-free culture medium (KSR)), and KSR-BN. FIG. 5a compares efficiencies of the chemically defined culture medium formulation iCD1 according to the present invention and other reported reprogramming culture schemes. The reprogrammed clones induced from 2 x 10⁴ embryonic fibroblasts in the above several culture schemes are counted at day 8 after infection. In the figure, the replications n in each group of test are 2, and the error bar represents standard deviation (SD). The result suggests that 1467.3 reprogrammed clones appear under iCD1 culture condition, while only 32.3, 20.7, and 68.3 reprogrammed cells respectively appear under the conditions of mES supplemented with vitamin C, mES to KSR, and KSR-BN. FIG. 5b shows the picture of the reprogrammed clones at day 8 after infection with the different culture schemes shown in FIG. 5a. The scale shown is 2 mm. The result suggests that the iCD1 culture system is obviously superior to any other culture system.

In order to more objectively compare the efficiencies under the several different culture conditions, the tests of the induced reprogramming efficiencies of KSR-BN, mES supplemented with vitamin C, and mES to KSR were repeated according to the report in literatures or patents, and the reprogramming efficiencies at different days are calculated. FIG. 5c shows that the KSR-BN culture scheme improves the reprogramming efficiency. Oct4-GFP MEFs were infected with Sox2/Klf4/Oct4 (SKO) viruses, and then cultured respectively in mES and KSR-BN culture media. The ratios of the reprogrammed cells in the two culture systems were detected at different time points through flow cytometry (FCM). The replications n in the test are 2, and the error bar represents standard deviation (SD). FIG. 5d shows that the mES improves the reprogramming efficiency after being supplemented with vitamin C. Oct4-GFP MEFs were infected with SKO viruses, and then cultured respectively in two culture media including mES and mES supplemented with vitamin C. The ratios of the reprogrammed cells in the two culture systems were detected at different time points through flow cytometry (FCM). The replications n in the test are 2, and the error bar represents standard deviation (SD). FIG. 5e shows that the mES->KSR culture scheme improves the reprogramming efficiency. Oct4-GFP MEFs were infected with SKO viruses, and then cultured respectively with the mES and mES to KSR culture schemes. The mES to KSR culture scheme is that before the first 4 days after infection, the mES culture medium is used, and then converted into the KSR culture medium. The ratios of the reprogrammed cells in the two culture systems were detected at different time points through flow cytometry (FCM). The replications n in the test are 2, and the error bar represents standard deviation (SD). The test results coincide with the report in literatures or patents. This result suggests that the comparison result of iCD1 with other several culture schemes is true and trustable.

### Example 3: Effects of ingredients in iCD1 on reprogramming

In order to evaluate the effects of main ingredients in iCD1 on the reprogramming efficiency, the iPS efficiency tests were carried out with iCD1 without vitamin C, bFGF, CHIR99021, lithium chloride, vitamin B12, and thiamine respectively or entirely, in which the iCD1 culture medium was used as a complete control, and the mES culture medium was used as a basic reference. The number of the reprogrammed clones at day 8 after infection are as shown in FIG. 6 a. FIG. 6a shows the effects of the six ingredients, bFGF, vitamin C, CHIR99021, lithium chloride, vitamin B12, and thiamine according to the present invention on the reprogramming efficiency. Oct4-GFP MEFs were infected with SKO viruses, and then cultured in a complete iCD1 culture medium, iCD1 culture media without a certain ingredient alone, iCD1 culture medium without all core ingredients, and the classical mES culture medium. Reprogrammed clones induced from 2 x 10⁴ cells under the different culture conditions were calculated respectively at day 8 after infection. In the figure, the replications n in each group of test are 3, and the error bar represents standard deviation (SD). Pictures of the reprogrammed clones at the same period are as shown in FIG. 6b. FIG. 6b shows reprogramming images at day 8 after infection under the different culture conditions shown in FIG. 6a, in which the scale shown is 2 mm. The results from the reprogrammed clone numbers at day 8 after infection and the pictures of the reprogrammed clones at the same period indicate that vitamin C, bFGF, CHIR99021, lithium chloride, vitamin B12, and thiamine are all have effects on the reprogramming efficiency, in which the effects of vitamin C, bFGF, and CHIR99021 on the reprogramming efficiency are especially remarkable. Almost no reprogrammed clones appear in absence of the six ingredients.

In order to determine whether the effects of the above six substances on reprogramming are cell growth dependent or independent, growth curves of the embryonic fibroblasts infected with reprogramming factor SKO and then cultured respectively in 9 culture states including iCD1, iCD1 without vitamin C, iCD1 without bFGF, iCD1 without CHIR99021, iCD1 without lithium chloride, iCD1 without vitamin B 12, iCD 1 without thiamine, iCD 1 without all of the six ingredients, and mES were plotted. 2 x 10⁴ initial Oct4-GFP MEFs were infected with SKO viruses, and then cultured respectively in the above 9 culture media. The cells were digested respectively at the day of infection and medium replacement (day D0) and days 3 and 6 after infection, and the cells per well were counted. The replications n in each group of test are 3, and the error bar represents standard deviation (SD). The result shown in FIG. 6c indicates that bFGF and chir99021 have different degrees of effects on cell growth, and Vc has no obvious effect on cell growth.

In order to further determine the optimum concentration of bFGF affecting reprogramming, different doses of bFGF were added to iCD1, to detect the effect on reprogramming. FIG. 7a shows dose response of the bFGF. 1 x 10⁴ Oct4-GFP MEFs were infected with SKO viruses, and then cultured respectively in iCD1 culture media supplemented with different doses of bFGF at a final concentration of 0-10 ng/ml. The reprogrammed clones were counted at day 10 after infection. The replications n in the test are 3, and the error bar represents standard deviation (SD). The result indicates that when the final concentration is higher than 5 ng/ml, the increase of the concentration of bFGF has no obvious improvement effect on the reprogramming efficiency. FIG. 7b shows the effect of the action time of bFGF. 1 x 10⁴ Oct4-GFP MEFs were infected with SKO viruses, and then cultured in an iCD1 culture medium to which bFGF was added respectively at different times from days 0 to 10. The fluorescent clones were calculated at day 10 after infection. The replications n in each group of test are 6, and the error bar represents standard deviation (SD). In the figure, the result of the test in which bFGF is added at different times in the reprogramming process indicates that under the iCD1 culture condition, bFGF plays a role mainly in the first 0-8 days of the reprogramming, and the prolongation of the action time has light effect on the further improvement of the reprogramming efficiency. Likely, in order to determine the optimum effect concentrations of vitamin C and the glycogen synthase kinase inhibitor CHIR99021 under modified iCD1 condition, different doses of vitamin C and the glycogen synthase kinase inhibitor CHIR99021 were respectively added to iCD1, to determine the most suitable effect concentrations thereof. FIG. 7c shows dose response of vitamin C. 2 x 10⁴ Oct4-GFP MEFs were infected with SKO viruses, and then cultured respectively in iCD1 culture media supplemented with different doses of vitamin C at a final concentration of 0-100 µg/ml. The reprogrammed clones were counted at day 10 after infection. The replications n in the test are 3, and the error bar represents standard deviation (SD). The result indicates that when the concentration of vitamin C or vitamin C-phosphate (a stable form of vitamin C) is higher than 25 µg/ml, the increase of the concentration of vitamin C has no obvious improvement effect on the reprogramming efficiency. FIG. 7d shows dose response of CHIR99021. 1 x 10⁴ Oct4-GFP MEFs were infected with SKO, and then cultured respectively in iCD1 culture media supplemented with different doses of CHIR99021 at a final concentration of 0-12 µmol/ml. The reprogrammed clones were counted at day 10 after infection. The replications n in the test are 3, and the error bar represents standard deviation (SD). The result indicates that the optimum effect concentration of the glycogen synthase kinase inhibitor is about 3 mM, and high dose of the glycogen synthase kinase inhibitor has an inhibition effect on the reprogramming efficiency instead. The above test results provide reference for working concentrations of the corresponding substances in the present invention.

In order to testify that other receptor tyrosine kinase family growth factors also play a role in reprogramming, epidermal growth factor (EGF) was also used in the reprogramming efficiency test. Oct4-GFP MEFs were infected with SKO viruses, and then cultured respectively in iCD1 culture media without receptor tyrosine kinase, supplemented with bFGF, and EGF. FIG. 7e shows a statistical result of the reprogrammed clones at day 10 after infection. The result indicates that besides bFGF, other receptor tyrosine kinases can also promote the reprogramming.

### Example 4: A83-01, valproic acid, and fetal bovine serum cannot further improve the reprogramming efficiency mediated by OKS

In order to further improve the iCD1 reprogramming efficiency, previously reported several substances able to improve the reprogramming efficiency, such as an ALK5 inhibitor A83-01, a histone methylase inhibitor valproic acid (VPA), and fetal bovine serum conventionally used for mouse embryonic stem cells, were added to iCD1 for the reprogramming efficiency test. FIG. 8a shows the number of the reprogrammed clones calculated at day 10 after the mouse fibroblasts are infected with SKO viruses, and then cultured respectively in chemically defined culture medium iCD1, and iCD1 culture media supplemented with A83-01 (0.5 µM), valproic acid (1 mM), and 2% fetal bovine serum. The result indicates that the ALK5 inhibitor A83-01, and the histone methylase inhibitor valproic acid (VPA) cannot further improve the iCD1 reprogramming efficiency, while the fetal bovine serum even has a certain degree of inhibition effect on the iCD1 reprogramming efficiency. FIG. 8b shows images of typical reprogrammed clones in the tests shown in FIG. 8a, in which the scale shown is 2 mm. The test result indicates that iCD1 is a very excellent culture system for inducing reprogramming.

### Example 5: Effect of initial seeding density on reprogramming efficiency

In order to discuss the effect of the initial cell density on the reprogramming efficiency, Oct4-GFP transgenic MEFs were subjected to two rounds of infection with SKO viruses, digested with trypsin, and seeded in a 96-well plate at different cell densities including specifically 5, 10, 50, 100, 200, 500, 1000, 2000, 5000, and 1 x 10⁴ cells/cm². After 8 days of culture in iCD1, the reprogramming efficiencies at various different seeding densities were calculated as a ratio the number of the reprogrammed clones at day 8 to the initial number of the seeded cells. The replications n in the test are 6, and the error bar represents standard deviation (SD). FIG. 9 shows the effect of the initial cell seeding density on the reprogramming efficiency. The test result indicates that when the initial cell density is from about 2000 to 5000 cells/cm², the reprogramming efficiency can reach a high value.

### Example 6: Under iCD1 culture condition, expression of the endogenous pluripotent molecular markers is activated rapidly, and the exogenous factor silences rapidly

In order to verify that the iCD1 culture system is superior to other culture system at a molecular level, relative expression levels of the pluripotent molecular marker Nanog and endogenous Oct4 specifically expressed by stem cells were used to evaluate the progress of the whole cell reprogramming process. Specific implementation method was as follows. Oct4-GFP transgenic MEFs were subjected to two rounds of infection with SKO viruses, and then cultured respectively in mES culture medium, KSR-BN culture medium, and iCD1 culture system. The day of infection and medium replacement was recorded as day 0, the culture samples were collected respectively after 2, 4, 6, and 8 days of culture in the mES culture medium, KSR-BN culture medium, and iCD1 culture medium, the ribonucleotides were extracted from the samples, and the expression levels of nanog and endogenous Oct4 were detected by real-time fluorescent quantitative polymerase chain reaction (RT-PCR). The test result is as shown in FIG. 10a, suggesting that under the iCD1 culture condition, the activation of nanog and endogenous Oc4 is obviously faster than that in the mES and KSR-BN groups. The replications n in each group of test are 3, and the error bar represents standard deviation (SD).

FIG. 10b shows the expression results of the pluripotent molecular markers verified at a protein level. Oct4-GFP MEFs were infected with SKO viruses, cultured in iCD1 for 8 consecutive days, and directly fixed on a cell culture plate for immunofluorescence assay. The result shows that at day 8, the pluripotent molecular markers Cdh1, SSEA-1, and Nanog are all expressed in the reprogrammed clones appeared in the plate. The scale shown is 100 microns. FIG. 10C shows expression profiles of Nanog, detected with an antibody by protein immunoblotting, in total protein extracted from culture harvested after 4 and 8 days of culture under the mES and iCD1 culture conditions. The test result indicates that at day 8 after infection, the expression of nanog under iCD1 culture condition is significant, and no expression of Nanog exists under the control mES condition. The result also further verifies that the reprogramming under iCD1 culture condition is significantly accelerated.

Silence of the exogenous transcription factor is an important event at the later stage of reprogramming, and also an index for determining complete reprogramming. In order to more intuitively tracking the silence of the exogenous transcription factor, red fluorescent protein Ds-Red was used to simulate the silence of the exogenous transcription factor. Specific implementation method was as follows. Oct4-GFP transgenic MEFs were infected with red fluorescent protein Ds-Red and SKO viruses, cultured in iCD1 culture medium, and tracked under a fluorescence microscope to observe the expression of green fluorescence (indicating the expression of the endogenous pluripotent molecular marker Oct4) and red fluorescence (indicating the expression of exogenous viruses). FIG. 11 shows reprogramming tracked in the iCD1 culture system. As shown in FIG. 11, at day 3 after infection, a part of cells begin to exhibit green fluorescence, and also express the red fluorescence at the same time, suggesting that the endogenous Oct4 gene has been activated, but the exogenous gene has not silenced. At day 6 after infection, the green fluorescence is significantly intensified, and the red fluorescence becomes weak, suggesting that the expression of endogenous Oct is significantly improved, and the exogenous viruses begin silence, which indicates that the cells begin to enter the later stage of the complete reprogramming. At day 8 after infection, most of clones express only reprogramming instead of red fluorescence, suggesting that the reprogramming process is substantially completed.

In order to further verify that the green fluorescence positive and red fluorescence negative cell population is more highly programmed than red and green fluorescence positive cell population and green fluorescence negative and red fluorescence positive cell population, the three cell populations in 8-day culture under the iCD1 culture condition were sorted out by a sorting flow cytometer. FIG. 12a shows the ratio of the reprogrammed cells (GFP) to the red fluorescent cells (DsRed) under the two conditions detected by flow cytometry (FCM). It can be seen from FIG. 12a that under the iCD1 culture condition, the ratio of the cell population entering reprogramming (green fluorescent cell population) is up to 54.3%, in which the ratio of the reprogramming positive and red fluorescence negative cell population is up to 34.8%. In contrast, in the mES culture system, only 0.3% of cells are reprogramming positive, and green fluorescence positive and red fluorescence negative cells are not detected. The ribonucleotides were extracted from the sorted three cell populations, and the expression of the pluripotent molecular markers nanog, Dppa3, and Rex1, and the endogenous Oct4, and the silence state of the exogenous Oct4 in the three cell populations were detected by real-time fluorescent quantitative PCR. The unsorted 8-day cultures in mES and iCD1 were used as overall controls. FIG. 12b shows expression profiles of the pluripotent molecular markers, detected by real-time fluorescent quantitative PCR, in different cell populations infected with SKO viruses and then cultured respectively in mES and iCD1 for 8 days, and the populations sorted from 8-day culture in iCD1. It can be seen from the result shown in FIG. 12b that in the three cell populations, the expression levels of the stem cell specific molecular markers in the green fluorescence positive and red fluorescence negative cell population are higher than those in other cell populations, and the silence of the exogenous viruses is also very complete. The expression levels of the stem cell specific pluripotent molecular markers in the green and red fluorescence positive cell population are in an intermediate state, and the exogenous Oct4 doesn't silence completely. The expression levels of the stem cell specific pluripotent molecular markers in the red fluorescence positive and green fluorescence negative cell populations are very low, and the expression level of exogenous Oct4 is very high. These test results strongly confirm that silence of the exogenous transcription factor is an important condition of complete reprogramming. It is also confirmed that the iCD 1 culture condition can accelerate the reprogramming process.

### Example 7: Test of complete reprogramming of MEFs at day 8 under iCD1 culture condition

Capability of forming chimeric mice is a very critical standard characteristic of mouse embryonic stem cells. In order to verify in a more strict sense that the reprogrammed clones have no difference from the embryonic stem cells, the reprogrammed cells picked through different methods were used in construction of chimeric mice. Specific method was as shown in FIG. 13a. Oct4-GFP transgenic MEFs were infected with mixed the three factors (SOX, KIF4 and OCT4) and DS-RED, and then cultured in the iCD1 culture medium. Cells were respectively picked at D8, D11, and D14 after infection with a machine or manually, to construct chimeric mice. Picking with machine includes digesting cells in original wells, spinning, and sorting out GFP positive and DS-RED negative cells through flow cytometry to construct chimeric mice. Manual picking includes peeling the clones from the attached wall at random under a fluorescence microscope, suspending the clones in a culture medium, collecting the culture medium containing the clone cluster to a 15 ml centrifugal tube after a certain amount of clone is peeled, spinning, collecting the clone pellet, washing with PBS, spinning to remove supernatant, adding 200 µl 0.25% trypsin, incubating at 37°C for 3-5 mins, terminating with serum, spinning, resuspending in the mES culture medium, and then constructing chimeric mice according to conventional chimeric mouse construction operations. FIG. 13b shows statistical table of the test shown in FIG. 13a implemented for different induction days. FIG. 13c shows chimeric mouse generated by injecting iPS cells obtained at day 8 into a blastocyst. The result strongly confirms that the reprogramming can be completed at day 8 under iCD 1 condition.

### Example 8: Effect of the culture medium of the present invention on reprogramming in presence of fewer factors

Under iCD1 culture condition, the extremely high reprogramming efficiency with the three SKO transcription factors indicates that reprogramming into somatic cells may be achieved with fewer transcription factors. Based on this assumption, different combinations of viruses (ok/os/sk) were used to infect mouse fibroblasts, and a specific method was as follows. OCT4 and KIF4 viruses were mixed at 1:1 (0.5 ml each), and infected 2 x 10⁴ cells/well in a 12-well plate; OCT4 and SOX2 viruses were mixed at 1:1 (0.5 ml each), and infected 3 x 10⁴ cells/well in a 12-well plate; and KIF and Sox2 viruses were mixed at 1:1 (0.5 ml each), and infected 3 x 10⁴ cells/well in a 12-well plate. The three groups of infected cells were cultured respectively in the iCD1 culture medium or the conventional mES culture medium, continuously cultured and observed. FIG. 14a shows primary clone picture and passage picture of the somatic cells reprogrammed with OK and OS under iCD1 culture condition. For the MEFs infected with OCT4 and KIF4 and cultured in the iCD1 culture system, 1-6 fluorescent clones per well appear in 12-15 days; for MEFs infected with OCT4 and SOX2 and cultured in the iCD1 culture system, 1-3 fluorescent clones per well appear in 18-25 days; and no clone appears under the conventional mES culture condition. In the wells where MEFs are infected with Kif4 and Sox2, no reprogrammed clone appears in 25 days under both the iCD1 and the mES culture conditions. FIG. 14b shows expressions of the stem cell specific molecular markers by iPS clones obtained by reprogramming with OK and OS. The picked OK and OS clones were subcultured on feeder cells, and it was found that pluripotent molecular markers such as Nanog and Rex1 were expressed by all the picked clones through immunofluorescence detection. FIG. 14c shows statistical results of the reprogramming test with Oct4/Kif4 and Oct4/Sox2. In the table, times for calculating the reprogrammed clones in each test and the statistical results thereof are shown. The three test results indicate that the test has good repeatability. FIG. 14d shows tissue sections of teratoma grown from nude mice injected with OK and OS clones. The obvious tissues of the three germ layers in the tissue sections indicate that the OK and OS clones have potential to differentiate to cells of the three germ layers.

### Example9: Oct4 can reprogram MEFs under iCD1 culture condition

The fact that the reprogrammed clones can be generated in the iCD1 with OK and OS suggests that Oct4 plays a key role in the reprogramming process, and thus attempts are made to investigate whether the reprogrammed clones can be generated in the iCD1 with Oct4 alone. 3 x 10⁴ Oct4-GFP transgenic MEFs were subjected to two rounds of infection with Oct4 viruses, and cultured in iCD1 for about 30 consecutive days. At this time, there was still no fluorescent clones appeared. The culture was digested with trypsin, subcultured on feeder cells, and continuously cultured in iCD1. At about day 5 after passage, green fluorescent reprogrammed clones appeared, as shown in FIG. 15a. The clones reprogrammed with OCT4 were picked and passaged, and the expressions of the pluripotent molecular markers were detected with an antibody through an immunofluorescence method. It was found that the clones all expressed pluripotent molecular markers such as nanog, SSEA-1, and Rex1, as shown in FIG. 15b. The expression levels of the endogenous pluripotent molecular markers Oct4, nanog, Dppa3, Rex1, and Dnmt31 relative to the mouse Oct4-GFP MEFs were detected with real-time fluorescent quantitative PCR. The result obtained from FIG. 15C also indicate that the expression levels of the endogenous Oct4, nanog, rex1, Dppa3, and Dnmt31 in the clones are similar to those in standard embryonic stem cells R1. FIG. 15d shows expression profile of the exogenous gene analyzed by real-time fluorescent quantitative PCR. Oct4-GFP MEFs at day 4 after being infected with SKO are used as positive control. The result shows that the exogenous transcription factor has been silenced in the picked reprogrammed clones. The test result of insertion assay (FIG. 15e) indicates that the picked clones are not caused by virus contamination. FIG. 15f shows chimeric mice generated by injecting Oct4 reprogrammed clones into a blastocyst. The generated chimeric male mouse and white strain mouse give birth to all-black mice, as shown in FIG. 15f. The result demonstrates that the Oct4 reprogrammed clones are germline chimeras. The above results strictly demonstrate that the Oct4 reprogrammed cells are similar to embryonic stem cells, and thus demonstrate that Oct4 can completely reprogram MEFs under the iCD1 culture condition.

### Example 10: Use of iCD1 culture medium in reprogramming with Oct4-vp16

Oct4-vp16 is Oct4 gene after which vp16 sequence is linked, co-expression thereof can enhance the transcription capability of Oct4. It is confirmed through experiments that the capability of Oct4-vp16, Sox2, and Kif4 for reprogramming somatic cells is higher than that of Oct4, Sox2, and Kif4. In order to verify that iCD1 is superior to other culture conditions in the reprogramming process in which Oct4-vp16 is involved, 2 x 10⁴ Oct4-GFP MEFs were infected with Oct4-vp16, Sox2, and Kif4, and then cultured respectively in mES, mES supplemented with vitamin C, and iCD1 culture system. At day 10 after infection, the reprogrammed clones were calculated. The replications n in each group of test are 3, and the error bar represents standard deviation (SD). The result is shown in FIG. 16a. It is found through the result that under iCD1 culture condition, 2500 reprogrammed clones appear at day 10, while 650 reprogrammed clones appear in the well containing mES supplemented with vitamin C, and only very few clones appear under mES condition. FIG. 16b shows fluorescent clone pictures under the three culture conditions. Similarly, the reprogramming capabilities of Oct4-vp16/Sox2, and Oct4-vp16/Kif4 were detected under iCD1 condition. Specific method was as follows. 3 x 10⁴ MEFs were respectively infected with Oct4-vp16/Sox2, and Oct4-vp16/Kif4 viruses, and cultured under iCD1 condition. The reprogrammed clones were calculated at different days. The replications n in each group of test are 3, and the error bar represents standard deviation (SD). The result shown in FIG. 17a indicates that under the iCD1 culture condition, Oct4-vp16/Sox2, and Oct4-vp16/Kif4 can reprogram the MEFs. At day 15 after infection, the reprogramming efficiency of Oct4-vp16/Sox2 is about 1%, and the reprogramming efficiency of Oct4-vp16/Kif4 is about 0.15%. FIG. 17b shows typical pictures of iPS clones induced with Oct4-vp16/Kif4 and Oct4-vp16/Sox2. The expressions of the pluripotent molecular markers were detected with an antibody through an immunofluorescence method, and it was found that the pluripotent molecular markers such as Nanog, Oct4, Rex1, and SSEA-1 were all expressed, as shown in FIG. 17c. FIG. 17c shows expressions of typical pluripotent molecular markers by iPS cells induced with Oct4-vp16K and Oct4-vp16S.

Reprogramming into adult stem cells can be achieved with Oct4 under iCD1 condition; however the efficiency is very low, and subculture is required. Whether Oct4-vp16 can improve the reprogramming efficiency of a single factor is highly concerned due to the properties of Oct4-vp16. In order to detect this, 3 x 10⁴ Oct4-GFP transgenic MEFs were infected with Oct4-vp16 viruses, and then cultured respectively in mES, mES supplemented with vitamin C, and iCD1 culture medium. As a result, it was found that at about day 10 after infection, green fluorescent reprogrammed clones appeared in the wells containing the iCD1 culture medium, as shown in FIG. 18a. No clones appeared in the wells containing mES and mES supplemented with vitamin C. At day 17 after infection, in the wells containing iCD1 medium, about 10 reprogrammed clones per well appeared, and no obvious reprogrammed clones appeared in the wells containing mES and mES supplemented with vitamin C. The statistical result is as shown in FIG. 18b. The picked clones were subcultured, and expressions of the pluripotent molecular markers were detected with an antibody through an immunofluorescent method. The result is as shown in FIG. 18c (FIG. 18c shows expressions of the pluripotent molecular markers by Oct4-vp16 induced clones), and indicates that the picked reprogrammed clones all express pluripotent molecular markers such as Nanog, and SSEA-1. The picked clones were injected into a blastocyst, and then implanted into uterus of a surrogate mouse. As a result, a chimeric mouse was successfully constructed, as shown in FIG. 18d. The result strictly demonstrates that the reprogrammed cells induced with Oct4-vp16 in iCD1 have similar pluripotency with the embryonic stem cells.

The above tests strongly demonstrate that in reprogramming by using Oct4-vp16, the iCD1 culture medium is obviously superior to other culture media.

### Example 11: Use of iCD1 core ingredients under mES condition

In order to test the use of the iCD1 core ingredients in serum, iCD1 core ingredients, vitamin C, lithium chloride, bFGF, insulin, and glycogen synthase kinase 3-b inhibitor CHIR99021 were added to mES, and a culture medium thus prepared is referred to as SmES (Super-mES). The reprogramming effects achieved with SKO and common Oct4 in SmES were detected. mES culture medium and mES culture medium supplemented with vitamin C were used as control. The result is as shown in FIG. 19a. At day 8 after infection with SKO, about 400 clones appeared in the well containing SmES media, while about 20 reprogrammed clones appear in the well containing mES supplemented with vitamin C. FIG. 19b shows a fluorescent picture of primary wells at day 8 under SmES culture condition. Similarly, the reprogramming effect achieved with a single factor Oct4 in SmES culture medium was also tested. In the SmES culture system, OCT4 can reprogram somatic cells. Oct4-GFP MEFs were infected with OCT4 viruses, and then culture respectively in mES, mES + vitamin C, and iCD1 culture medium, and the fluorescent clones were observed at different days. FIG. 19c shows the results of three independent tests. The results suggest that under SmES condition, Oct4 reprograms MEFs. FIG. 19d shows pictures of primary clones and passage clones.

## Claims

1. A culture medium supplement, **characterized by** comprising vitamin C and a glycogen synthase kinase-3 inhibitor.

2. The culture medium supplement according to claim 1, **characterized in that** the glycogen synthase kinase-3 inhibitor is at least one of lithium ion, Chir99021, BIO, and SB216763.

3. The culture medium supplement according to claim 1, **characterized by** further comprising vitamin B12, insulin, a receptor tyrosine kinase, and an anti-oxidant.

4. The culture medium supplement according to claim 3, **characterized in that** the receptor tyrosine kinase is at least one of basic fibroblast growth factor(bFGF), epidermal growth factor (EGF), vascular endothelial growth factor (VEGF), platelet-derived growth factor (PDGF), insulin-like growth factor (IGF), and hepatocyte growth factor (HGF).

5. The culture medium supplement according to claim 3, **characterized in that** the anti-oxidant is at least one of thiamine, superoxide dismutase, catalase, reduced glutathione, vitamin E, acetylated vitamin E, linoleic acid, linolenic acid, and sodium selenite.

6. The culture medium supplement according to claim 1 or 3, **characterized by** further comprising a serum replacement cell growth promoter.

7. The culture medium supplement according to claim 6, **characterized in that** the serum replacement cell growth promoter is at least one of a albumin hydrolyzate, transferrin, triiodothyronine, adrenalone, lipoic acid, ethanolamine, progesterone, putrescine, and vitamin A.

8. A complete culture medium, **characterized by** being formed by one or more of a basal culture medium, serum, a serum replacement supplement, and the culture medium supplement according to claim 1, 3, or 6.

9. A complete culture medium, **characterized by** being formed by a basal culture medium and the culture medium supplement according to claim 1, 3, or 6.

10. Use of the culture medium supplement according to claim 1, 3, or 6, or the complete culture medium according to claim 8 or 9 in culture of induced pluripotent stem (iPS) cells or eukaryotic cells.
